# FASCICULE DE BREVET EUROPEEN

(11) **EP 1 745 786 B1**
(45) Date de publication et mention de la délivrance du brevet: **01.01.2014**
(21) Numéro de dépôt: 06291167.2
(22) Date de dépôt: 18.07.2006
(51) Int. Cl.: A61K 31/536, A61K 31/517, A61P 25/00

(54) **Composés neuroprotecteurs et compositions pharmaceutiques les comprenant**
Nervenschützende Verbindungen und pharmazeutische Zusammensetzungen, die sie enthalten
Neuroprotective compounds and pharmaceutical compositions comprising them

(30) Priorité: 19.07.2005 FR 0507648
(43) Date de publication de la demande: 24.01.2007
(73) Titulaire: Biocodex, 94250 Gentilly (FR)
(72) Inventeur: Verleye, Marc, 60190 Remy (FR); Le Guern, Marie-Emmanuelle, 60200 Compiegne (FR); Girard, Philippe, 60280 Margny-Les-Compiegne (FR); Gillardin, Jean-Marie, 60680 Jonquieres (FR); Berthon-Cedille, Laurence, 60490 Ricquebourg (FR); Hublot, Bernard, 60200 Compiegne (FR)
(74) Mandataire: Vial, Lionel

(56) Documents cités:
- WO-A-2004/035000
- DE-A1- 3 439 055
- US-A1- 2004 209 904
- DATABASE EMBASE [Online] ELSEVIER SCIENCE PUBLISHERS, AMSTERDAM, NL; 1985, KRUSE H J ET AL: "Etifoxine: Evaluation of its anticonvulsant profile in mice in comparison with sodium valproate, phenytoin and clobazam" XP002372434 Database accession no. EMB-1985045795 & ARZNEIMITTEL-FORSCHUNG/DRUG RESEARCH 1985 GERMANY, vol. 35, no. 1, 1985, pages 133-135,
- DATABASE EMBASE [Online] ELSEVIER SCIENCE PUBLISHERS, AMSTERDAM, NL; 1986, KRUSE H J ET AL: "Potentiation of clobazam's anticonvulsant activity by etifoxine, a non-benzodiazepine tranquilizer, in mice. Comparison studies with sodium valproate" XP002372435 Database accession no. EMB-1986230303 & ARZNEIMITTEL-FORSCHUNG/DRUG RESEARCH 1986 GERMANY, vol. 36, no. 9, 1986, pages 1320-1322,
- DOONGAJI D R ET AL: "A preliminary study of etafenoxine" JOURNAL OF POSTGRADUATE MEDICINE. JAN 1976, vol. 22, no. 1, janvier 1976 (1976-01), pages 37-43, XP009063584 ISSN: 0022-3859
- GRATTON L ET AL: "Proceedings: etafenoxin in the treatment of neurosis--an uncontrolled clinical study" PSYCHOPHARMACOLOGY BULLETIN. JAN 1976, vol. 12, no. 1, janvier 1976 (1976-01), pages 16-18, XP009063592 ISSN: 0048-5764

## Description

La présente invention concerne l'utilisation de composés neuroprotecteurs dans le cadre du traitement des détériorations neuronales liées à des pathologies du système nerveux.

La détérioration, notamment la mort, neuronale joue un rôle essentiel dans pratiquement toutes les pathologies impliquant une dégénération neuronale aiguë ou chronique. On pense que de nombreux modulateurs neurochimiques interviennent dans la survenue de lésions du système nerveux. Par exemple, dans l'épilepsie, la neurotransmission avec excès de glutamate, l'altération de l'inhibition liée au GABA et les altérations de l'équilibre acido-basique peuvent entraîner une série d'évènements conduisant à des altérations neuronales et à la mort cellulaire.

Plusieurs voies biochimiques menant à la mort neuronale ont pu être élucidées ces dernières années. Un des cas les plus documentés concerne l'excitotoxicité du glutamate. Cet acide aminé neurotransmetteur est libéré de manière excessive en cas d'ischémie par exemple, ce qui provoque, *via* une activation excessive de ses récepteurs neuronaux, un afflux d'ions calcium à l'intérieur des neurones et conduit à la mort cellulaire par nécrose ou apoptose.

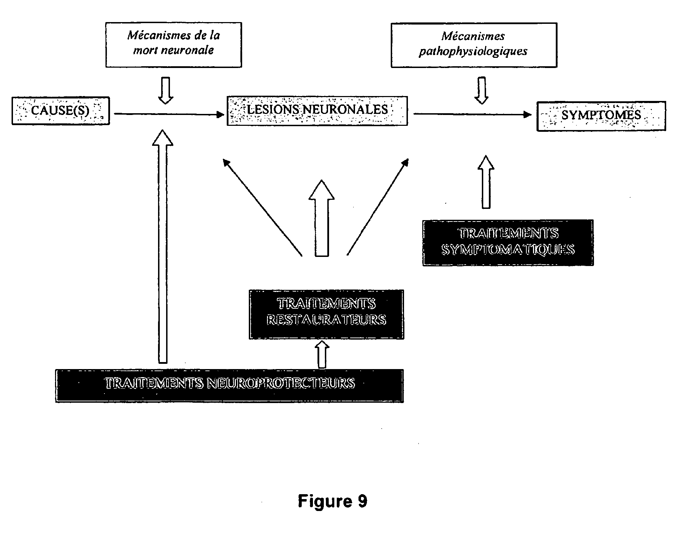

Par définition, la neuroprotection est une activité ayant pour conséquence la préservation, la récupération, la guérison ou la régénération du système nerveux, de ses cellules, sa structure et son fonction (Vajda et al. (2002) J Clin Neurosci.9:4-8). La neuroprotection s'exerce notamment, comme indiqué sur la **Figure 9****,** sur les processus conduisant à la mort neuronale.

Les mécanismes présumés, à la fois complexes et variés, de la mort neuronale, tels que le stress oxydatif, les dysfonctionnements mitochondriaux, l'agrégation protéique, l'apoptose et l'inflammation (Youdim et al. (2005), TIPS 26:27-35), laissent penser que les traitements neuroprotecteurs agissent à plusieurs niveaux sur le plan neurologique et biochimique (Youdim MB et al. (2005) J. Neural. Transm. 112:519-537) (Sellal et al. (2005) Therapie, 60:89-107).

Ainsi, dans le cadre du traitement des accidents ischémiques cérébraux, il a pu être montré que de nombreux agents inhibant certaines étapes du processus de mort neuronale, tels que des antagonistes du glutamate, des agents anti-inflammatoires, des agents modulateurs des canaux ioniques, des agents anti-radicaux libres, ou encore des antagonistes du GABA, possédaient une action neuroprotectrice chez l'animal.

Toutefois, les nombreux essais cliniques menés à ce jour n'ont pas permis de confirmer le potentiel de ces composés en tant qu'agents de neuroprotection chez l'homme.

Un objet de la présente invention est donc de fournir des compositions pharmaceutiques ayant une efficacité neuroprotectrice supérieure à celle des compositions déjà connues.

La demande de brevet DE 34 39 055 indique que l'étifoxine présenterait des effets anxiolytiques et anticonvulsivants, c'est-à-dire le profil d'un antiépileptique avec une composante tranquillisante. Cette demande indique également que l'étifoxine potentialiserait, dans un modèle animal, les effets antiépileptiques et anxiolytiques du clobazam.

L'abrégé de l'article de Kruse & Kuch (1985) Arzneimittel-Forschung/Drug Research 35:133-135 indique que l'étifoxine présenterait des effets anticonvulsivants chez la souris.

L'article de Doongaji et al. (1976) Journal of Postgraduate Medicine 22:37-43 indique, quant à lui, que l'étafénoxine serait utile pour le traitement de symptômes psychiques et somatiques de l'anxiété accompagnée de dépression.

La présente invention découle en particulier de la mise en évidence par les Inventeurs que l'étifoxine et la déséthyl-étifoxine possèdent une action neuroprotectrice in vitro et in vivo chez l'animal.

L'invention est définie par les revendications 1 à 19 ci-après.

La présente invention concerne ainsi l'utilisation d'au moins un composé de formule (I) suivante : 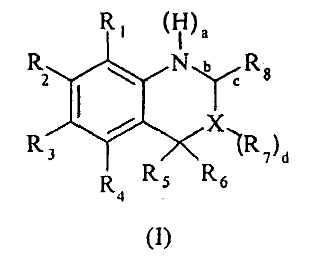 dans laquelle :
- a représente 0 ou 1 ;
- b représente une simple liaison ou une double liaison ;
- c représente une simple liaison ou une double liaison ;
- d représente 0 ou 1 ;
- X représente un atome d'oxygène ou d'azote, sous réserve que lorsque X représente un atome d'oxygène alors d vaut 0 et que lorsque X représente un atome d'azote alors d vaut 1 ;
- R₁, R₂, R₃, et R₄, identiques ou différents, représentent un atome d'hydrogène, un atome d'halogène, notamment choisi parmi F, Cl, Br, ou I, un groupe hydroxyle, ou un groupe alkoxyle de 1 ou 2 atomes de carbone ;
- R₅ et R₆, identiques ou différents, représentent un atome d'hydrogène, un groupe alkyle ou cycloalkyle de 1 à 6 atomes de carbone, ou un groupe aryle de 6 atomes de carbone dont le noyau aromatique est éventuellement substitué par un ou plusieurs atomes d'halogène ou un ou plusieurs groupes hydroxyle, alkoxyle de 1 ou 2 atomes de carbone, trifluorométhyle ou nitro ;
- R₇ représente un atome d'hydrogène, un groupe hydroxyle, ou un groupe alkyle ou hydroxyalkyle de 1 à 3 atomes de carbone ;
- R₈ représente un atome d'oxygène ou un groupe -NR₉R₁₀, R₉ et R₁₀, identiques ou différents, représentant un atome d'hydrogène, un groupe hydroxyle, ou un groupe alkyle ou hydroxyalkyle de 1 à 3 atomes de carbone, sous réserve que lorsque R₈ représente un atome d'oxygène alors a vaut 1, b représente une simple liaison et c représente une double liaison et que lorsque R₈ représente un groupe -NR₉R₁₀ alors a vaut 0, b représente une double liaison et c représente une simple liaison ;
ou de ses sels pharmaceutiquement acceptables,
pour la préparation d'un médicament à activité neuroprotectrice destiné au traitement des détériorations neuronales.

On désigne par « activité neuroprotectrice » une action ayant pour conséquence la préservation, la récupération, la guérison ou la régénération du système nerveux, de ses cellules, de sa structure et de sa fonction (Vajda et al. (2002) J Clin Neurosci 9:4-8).

On désigne par « détériorations neuronales », les lésions microscopiques des cellules observées dans différentes pathologies neurologiques. Ces lésions peuvent notamment être de type ischémique, atrophique, perte neuronale, inclusions intracytoplasmiques ou intranucléaires, dégénérescence neurofibrillaire ou granulo-vacuolaire.

A titre d'exemple, les lésions neuronales observées lors de la maladie d'Alzheimer associent une dégénérescence neurofibrillaire avec une perte de synapses dans l'hippocampe et les régions voisines du lobe temporal, des foyers intracorticaux de prolongements neuronaux épaissis à la fois axonaux et dendritiques (neurites), et une dégénérescence granulovacuolaire. Les lésions ischémiques observées lors d'accidents vasculaires ischémiques associent un noyau foncé et un cytoplasme très basophile et rétracté. Les maladies neurodégénératrices s'accompagnent de lésions à type d'atrophie neuronale avec dépopulation de zones caractéristiques de la maladie (Augustinack et al. (2002) ; Acta Neuropatho1.103:26-35) - (Harrison - Arnett Blackwell Ed 1995) - (Cambier - Masson Ed 2000).

Dans un mode de réalisation particulier de l'utilisation telle que définie ci-dessus, les détériorations neuronales sont liées aux pathologies choisies dans la liste comprenant :
- l'épilepsie,
- les accidents vasculaires cérébraux, ischémiques ou hémorragiques,
- les maladies neurodégénératives, telles que la maladie de Charcot-Marie-Tooth ou la Maladie de Friedreich,
- les phacomatoses, notamment les neurofibromatoses,
- les neuropathies, telles que les neuropathies carentielles, notamment alcooliques, les neuropathies toxiques ou médicamenteuses, notamment à la vincristine, les neuropathies liées à une affection métabolique, telle que le diabète, les neuropathies liées à un processus inflammatoire, notamment le syndrome de Guillain-Barré, les neuropathies infectieuses, notamment le zona, et les neuropathies radiques,
- la polynévrite paranéoplasique,
- la sclérose en plaque,
- la sclérose latérale amyotrophique,
- la schizophrénie,
- la dépression,
- les tumeurs cérébrales,
- la maladie de Parkinson, et
- les démences, telles que la maladie d'Alzheimer, la maladie de Pick ou la démence vasculaire.

La synthèse des composés de formule (I) définis ci-dessus peut être aisément mise en oeuvre à partir des enseignements du brevet français n°1 571 287.

Les sels pharmaceutiquement acceptables selon l'invention apparaîtront de manière évidente pour l'homme du métier, en particulier les sels de chlorhydrate des composés de formule (I) selon l'invention sont préférés.

Comme on l'entend ici, la présente invention concerne également l'utilisation, telle que définie ci-dessus, des formes optiquement actives du composé de formule (I), tels que les énantiomères suivants (lorsque R₅ et R₆ sont différents) : 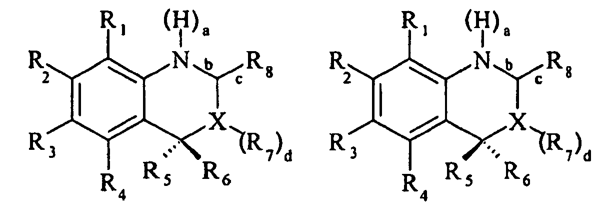 ou leurs mélanges, en particulier leur mélange racémique.

Dans un mode de réalisation particulier, l'invention concerne l'utilisation telle que définie ci-dessus d'un composé de formule (VIII) suivante : 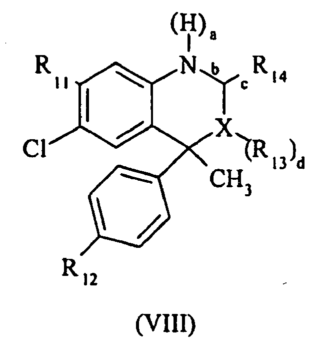 dans laquelle :
- a représente 0 ou 1 ;
- b représente une simple liaison ou une double liaison ;
- c représente une simple liaison ou une double liaison ;
- d représente 0 ou 1 ;
- X représente un atome d'oxygène ou d'azote, sous réserve que lorsque X représente un atome d'oxygène alors d vaut 0 et que lorsque X représente un atome d'azote alors d vaut 1 ;
- R₁₁ et R₁₂ identiques ou différents représentent -H ou -OH ;
- R₁₃ représente -H ou un groupe -CH₂-CH₃ ;
- R₁₄ représente un atome d'oxygène ou un groupe -NH₂ ou -NH-CH₂-CH₃, sous réserve que lorsque R₁₄ représente un atome d'oxygène alors a vaut 1, b représente une simple liaison et c représente une double liaison et que lorsque R₁₄ représente un groupe -NH₂ ou -NH-CH₂-CH₃ alors a vaut 0, b représente une double liaison et c représente une simple liaison.

La présente invention concerne également l'utilisation, telle que définie ci-dessus, des formes optiquement actives du composé de formule (VIII), tels que les énantiomères suivants : 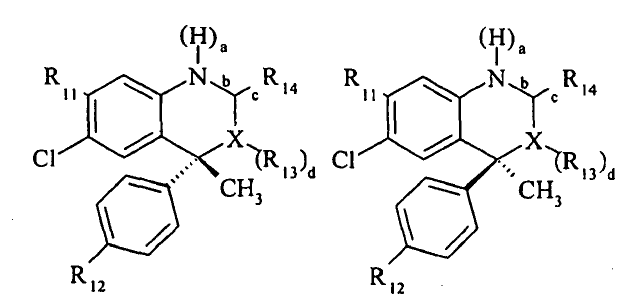 ou leurs mélanges, en particulier leur mélange racémique.

Dans un mode de réalisation particulier, l'invention concerne l'utilisation telle que définie ci-dessus, d'un composé de formule (II) suivante : 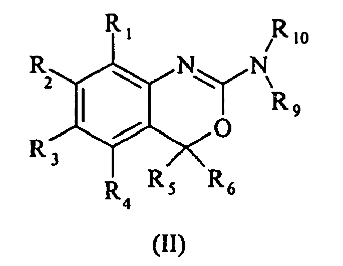 dans laquelle R₁, R₂, R₃, R₄, R₅, R₆, R₉ et R₁₀ sont tels que définis ci-dessus.

La présente invention concerne également l'utilisation, telle que définie ci-dessus, des formes optiquement actives du composé de formule (II), tels que les énantiomères suivants (lorsque R₅ et R₆ sont différents) : 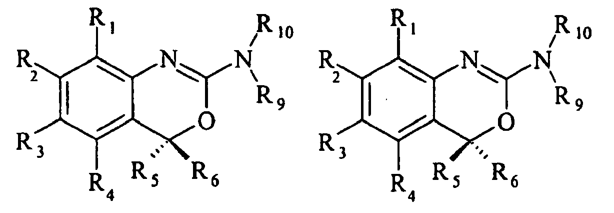 ou leurs mélanges, en particulier leur mélange racémique.

Dans un autre mode de réalisation particulier, l'invention concerne l'utilisation telle que définie ci-dessus, des composés de formules (III) et (IV) suivantes :

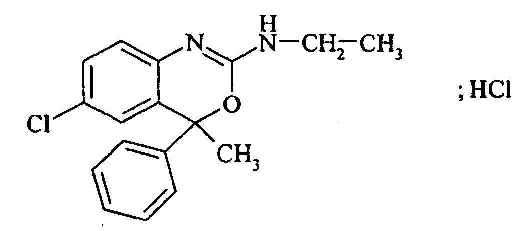

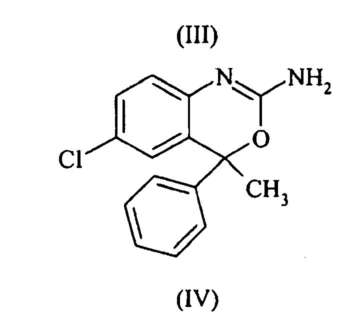

Le composé de formule (III) est l'étifoxine, ou chlorhydrate de 6-chloro-2-éthylamino-4-méthyl-4-phényl-4H-[3,1]benzoxazine.

Le composé de formule (IV), la déséthyl-étifoxine ou 2-amino-6-chloro-4-méthyl-4-phényl-4H-[3,1]benzoxazine, est un métabolite de l'étifoxine.

La présente invention concerne également l'utilisation, telle que définie ci-dessus, des formes optiquement actives du composé de formule (III), tels que les énantiomères suivants : 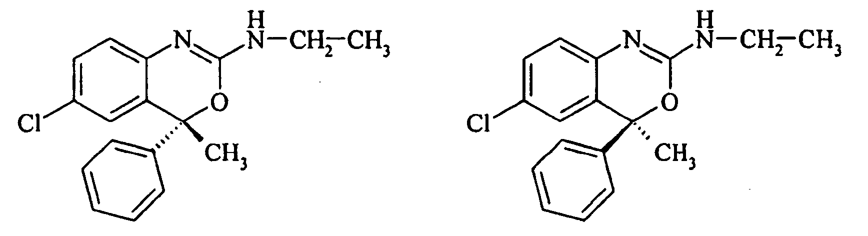 ou leurs mélanges, en particulier leur mélange racémique, notamment sous forme chlorhydrate, ainsi que l'utilisation, telle que définie ci-dessus, des formes optiquement actives du composé de formule (IV), tels que les énantiomères suivants : 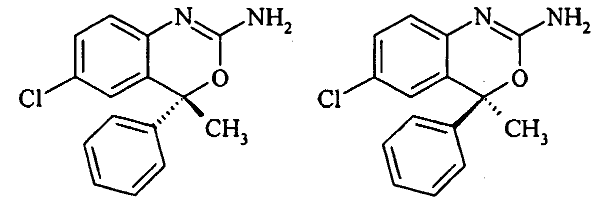 ou leurs mélanges, en particulier leur mélange racémique.

Dans un autre mode de réalisation, l'invention concerne l'utilisation telle que définie ci-dessus, d'un composé de formule (V) suivante : 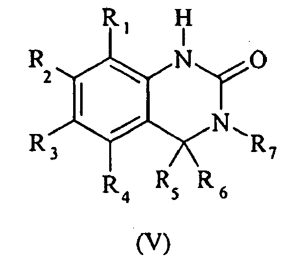 dans laquelle R₁, R₂, R₃, R₅, R₆, et R₇ sont tels que définis ci-dessus.

La présente invention concerne également l'utilisation, telle que définie ci-dessus, des formes optiquement actives du composé de formule (V), tels que les énantiomères suivants (lorsque R₅ et R₆ sont différents) : 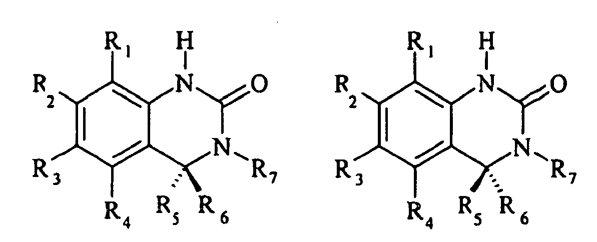 ou leurs mélanges, en particulier leur mélange racémique.

Dans un autre mode de réalisation particulier, l'invention concerne l'utilisation telle que définie ci-dessus, de composés de formules (VI) et (VII) suivantes :

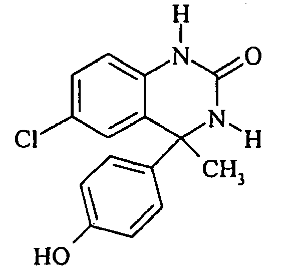

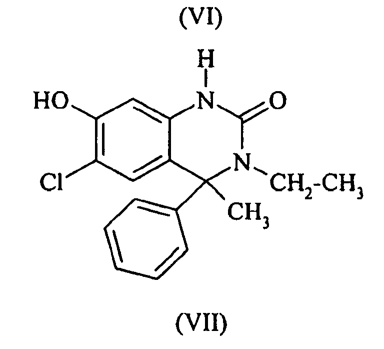

Les composés de formule (VI) (6-Chloro-4-(4-hydroxy-phényl)-4-méthyl-3,4-dihydro-1H-quinazolin-2-one) et (VII) (6-chloro-3-éthyl-7-hydroxy-4-méthyl-4-phényl-3,4-dihydro-1H-quinazolin-2-one) sont des métabolites de l'étifoxine.

La présente invention concerne également l'utilisation, telle que définie ci-dessus, des formes optiquement actives du composé de formule (VI), tels que les énantiomères suivants : 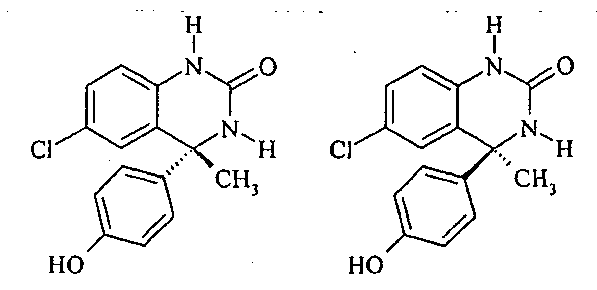 ou leurs mélanges, en particulier leur mélange racémique, ainsi que l'utilisation, telle que définie ci-dessus, des formes optiquement actives du composé de formule (VII), tels que les énantiomères suivants : 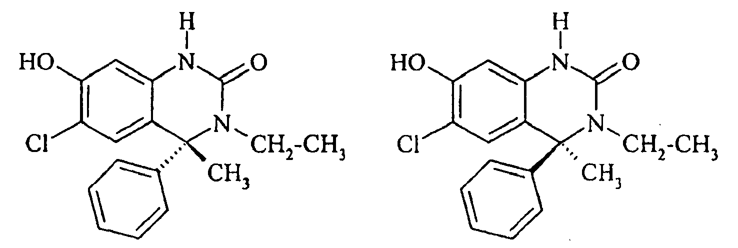 ou leurs mélanges, en particulier leur mélange racémique.

Dans un mode de réalisation particulier de l'invention, le médicament défini ci-dessus convient pour l'administration à un individu en ayant besoin d'une dose unitaire d'environ 50 mg à environ 1500 mg, notamment d'environ 150 à 200 mg du composé tel que défini ci-dessus.

Dans un autre mode de réalisation particulier de l'invention, le médicament défini ci-dessus convient pour l'administration à un individu en ayant besoin d'une dose d'environ 50 mg/j à environ 1500 mg/j, notamment d'environ 150 mg/j à environ 200 mg/j, du composé tel que défini ci-dessus.

Selon un mode de réalisation préféré de l'invention, le médicament défini ci-dessus convient pour une administration par voie orale.

Selon un autre mode de réalisation préféré de l'invention, le médicament défini ci-dessus se présente sous la forme d'une poudre, de cachets, de gélules ou de sachets.

Dans un mode de réalisation particulier de l'invention, le composé défini ci-dessus est associé à au moins un composé additionnel destiné à la prévention ou au traitement des pathologies définies ci-dessus.

La présente invention concerne également des produits contenant :
- au moins un composé de formule (I) tel que défini ci-dessus ou ses sels pharmaceutiquement acceptables, et
- au moins un composé additionnel destiné à la prévention ou au traitement des pathologies choisies dans la liste comprenant :
   - l'épilepsie,
   - les accidents vasculaires cérébraux, ischémiques ou hémorragiques,
   - les maladies neurodégénératives, telles que la maladie de Charcot-Marie-Tooth ou la Maladie de Friedreich,
   - les phacomatoses, notamment les neurofibromatoses,
   - les neuropathies, telles que les neuropathies carentielles, notamment alcooliques, les neuropathies toxiques ou médicamenteuses, notamment liées à l'administration de vincristine, les neuropathies liées à une affection métabolique, telle que le diabète, les neuropathies liées à un processus inflammatoire, notamment le syndrome de Guillain-Barré, les neuropathies infectieuses, notamment le zona, et les neuropathies radiques,
   - la polynévrite paranéoplasique,
   - la sclérose en plaque,
   - la sclérose latérale amyotrophique,
   - la schizophrénie,
   - la dépression,
   - les tumeurs cérébrales,
   - la maladie de Parkinson, et
   - les démences, telles que la maladie d'Alzheimer, la maladie de Pick ou la démence vasculaire,
comme produit de combinaison pour une utilisation séparée, simultanée ou étalée dans le temps pour la prévention ou le traitement des détériorations neuronales liées à ces pathologies.

Dans un mode de réalisation préféré de l'invention le composé additionnel défini ci-dessus est choisi dans la liste comprenant :
- un antiépileptique, notamment :
   - Acide valproïque,
   - Barbituriques,
   - Carbamazépine,
   - Ethosuximide,
   - Gabapentine,
   - Lamotrigine,
   - Stiripentol,
   - Tiagabine,
   - Vigabatrin,
- un antiparkinsonien, notamment :
   - dopaminergique, tel que :
      → Lévodopa associée à inhibiteur de la dopadécarboxylase,
      → Agonistes dopaminergiques,
   - anticholinergique, tel que :
      → Bipéridène,
      → Trihexiphénidyle,
      → Tropatépine,
   - un inhibiteur de la monoamine oxydase B (IMAO B),
   - un inhibiteur de la catechol-O-méthyl transférase (COMT),
- un composé destiné au traitement de la maladie d'Alzheimer, notamment :
   - un anticholinestérasique, tel que :
      → Donépézil,
      → Galantamine,
      → Rivastigmine,
   - un antagoniste des récepteurs NMDA, tel que :
      → Mémantine,
- un composé destiné au traitement des AVC (accidents vasculaires cérébraux) ischémiques en phase aiguë (Altéplase) et de leurs suites (Dihydroergocristine, Piracétam), notamment selon le type d'AVC :
   → Héparine,
   → Aspirine,
   → Nicardipine.
- un composé destiné au traitement de la sclérose en plaque, notamment :
   - Interféron β,
   - Glatiramère acétate,
   - Mitoxantrone,
- un composé destiné au traitement de la sclérose latérale amyotrophique, notamment :
   - Riluzole,
- un composé destiné au traitement du syndrome de Guillain Barré, notamment :
   - Immunoglobulines humaines de type G,
   - Tegeline,
- un composé destiné au traitement des hémorragies cérébrales, notamment :
   - Nimodipine,
- un antidépresseur, notamment :
   - un imipraminique, tel que :
      → Anafranil,
      → Clomipramine,
      → Amoxapine,
      → Amitriptyline,
   - un inhibiteur sélectif de la recapture de la sérotonine, tel que :
      → Fluoxétine,
      → Paroxétine,
      → Citalopram,
      → Fluvoxamine,
   - un inhibiteur sélectif de la recapture de la sérotonine et de la noradrénaline, tel que :
      → Venlafaxine,
      → Mirtazapine,
      → Tianeptine,
- un composé destiné au traitement de la schizophrénie, notamment :
   - un psychotrope, tel que :
      → Olanzapine,
      → Rispéridone,
      → Clozapine,
   - un neuroleptique, tel que
      → Halopéridol,
      → Pipotiazine,
- un composé destiné au traitement des tumeurs cérébrales, notamment un agent de chimiothérapie,
- un composé destiné au traitement de la neuropathie diabétique, notamment un composé destiné à la correction du trouble métabolique (insuline),
- un composé destiné au traitement de la polynévrite alcoolique, notamment la vitamine B1.

Avantageusement, le composé de formule générale (I) ou ses sels pharmaceutiquement acceptables, sont utilisés comme adjuvant destiné à augmenter les effets de composés destinés au traitement des pathologies définies ci-dessus, tel que les composés additionnels définis ci-dessus.

### DESCRIPTION DES FIGURES

**Figure 1**

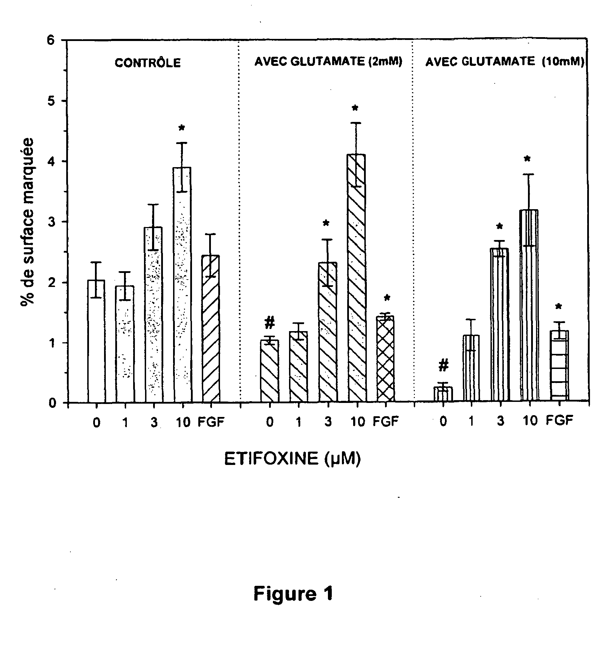

   La Figure 1 représente les effets de concentrations croissantes d'étifoxine (en abscisses, µM) sur la densité de marquage des prolongements neuritiques (en ordonnées, pourcentage de surface marquée) en absence (contrôle) ou en présence de glutamate (2 ou 10 mM) après 6 heures d'incubation. Chaque colonne représente la moyenne ± écart standard à la moyenne (8 mesures par groupe). FGF représente le facteur de croissance. Le signe étoile (*) représente p<0,05 par rapport aux témoins respectifs (dose 0) et le signe dièse (#) représente p<0,05 par rapport aux contrôles (sans glutamate).
**Figure 2**

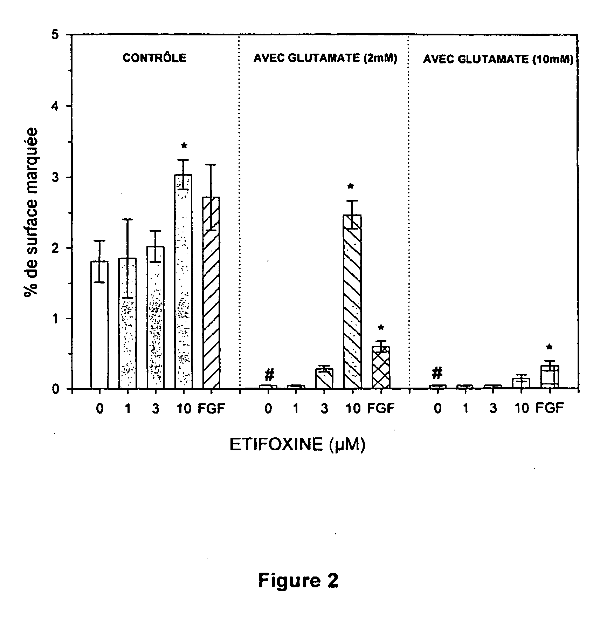

   La Figure 2 représente les effets de concentrations croissantes d'étifoxine (en abscisses, µM) sur la densité de marquage des prolongements neuritiques (en ordonnées, pourcentage de surface marquée) en absence (contrôle) ou en présence de glutamate (2 ou 10 mM) après 24 heures d'incubation. Chaque colonne représente la moyenne ± écart standard à la moyenne (8 mesures par groupe). FGF représente le facteur de croissance. Le signe étoile (*) représente p<0,05 par rapport aux témoins respectifs (dose 0) et le signe dièse (#) représente p<0,05 par rapport aux contrôles (sans glutamate).
**Figure 3**

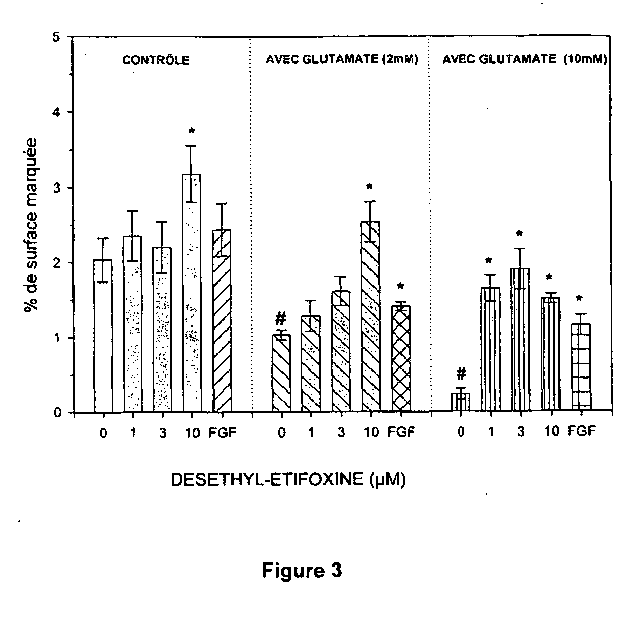

   La Figure 3 représente les effets de concentrations croissantes de déséthyl-étifoxine (en abscisses, µM) sur la densité de marquage des prolongements neuritiques (en ordonnées, pourcentage de surface marquée) en absence (contrôle) ou en présence de glutamate (2 ou 10 mM) après 6 heures d'incubation. Chaque colonne représente la moyenne ± écart standard à la moyenne (8 mesures par groupe). FGF représente le facteur de croissance. Le signe étoile (*) représente p<0,05 par rapport aux témoins respectifs (dose 0) et le signe dièse (#) représente p<0,05 par rapport aux contrôles (sans glutamate).
**Figure 4A et Figure 4B**

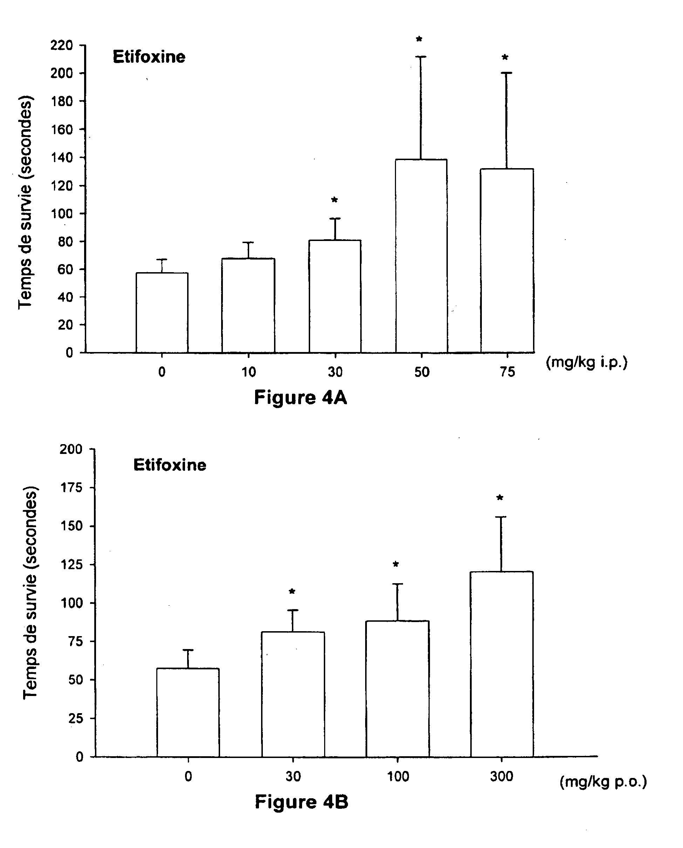

   Les Figures 4A et 4B représentent les effets de concentrations croissantes d'étifoxine (en abscisses, mg/kg) administrées par voie intrapéritonéale (Figure 4A) ou par voie orale (Figure 4B) sur le temps de survie (en ordonnées, en secondes, moyenne ± écart standard à la moyenne) de souris soumises à une hypoxie hypobare induite par une diminution de la pression atmosphérique à 160 mmHg (9 ou 10 animaux par groupe). Le signe étoile (*) représente p<0,05 par rapport aux témoins respectifs, dans le test statistique ANOVA.
**Figure 5A et Figure 5B**

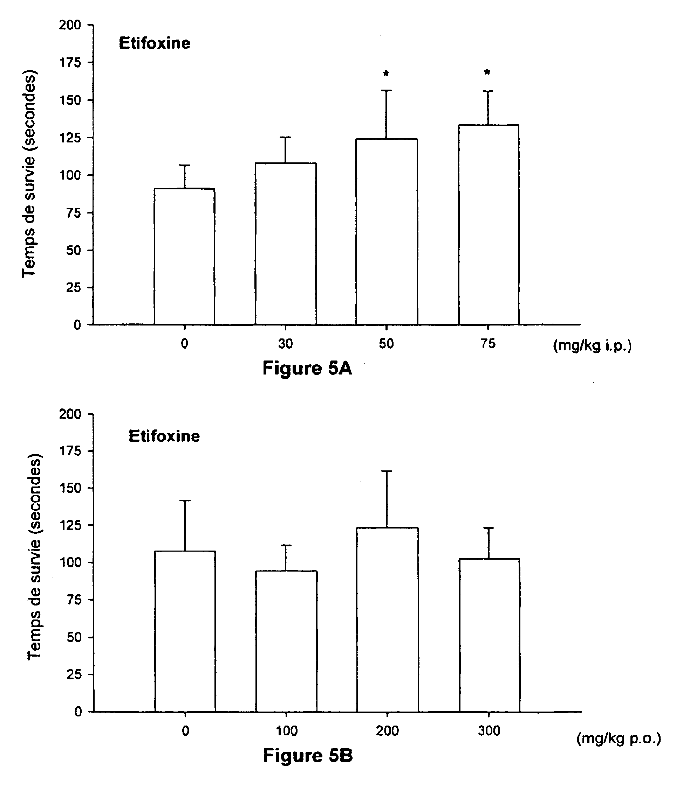

   Les Figures 5A et 5B représentent les effets de concentrations croissantes d'étifoxine (en abscisses, mg/kg) administrées par voie intrapéritonéale (Figure 5A) ou par voie orale (Figure 5B) sur le temps de survie (en ordonnées, en secondes, moyenne ± écart standard à la moyenne) de souris soumises à une hypoxie histotoxique induite par une administration intrapéritonéale de 15 mg/kg de cyanure de potassium (9 à 19 animaux par groupe). Le signe étoile (*) représente p<0,05 par rapport aux témoins respectifs, dans le test statistique ANOVA.
**Figure 6A et Figure 6B**

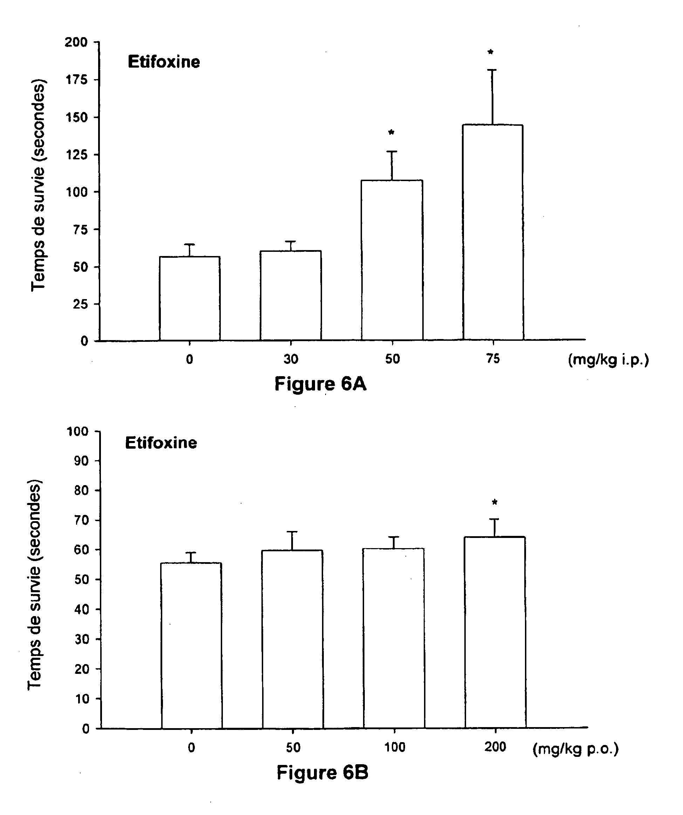

   Les Figures 6A et 6B représentent les effets de concentrations croissantes d'étifoxine (en abscisses, mg/kg) administrées par voie intrapéritonéale (Figure 6A) ou par voie orale (Figure 6B) sur le temps de survie (en ordonnées, en secondes, moyenne ± écart standard à la moyenne) de rats soumis à une hypoxie histotoxique induite par une administration intraveineuse de 4 mg/kg de cyanure de potassium (9 ou 10 animaux par groupe). Le signe étoile (*) représente p<0,05 par rapport aux témoins respectifs, dans le test statistique ANOVA.
**Figure 7A et Figure 7B**

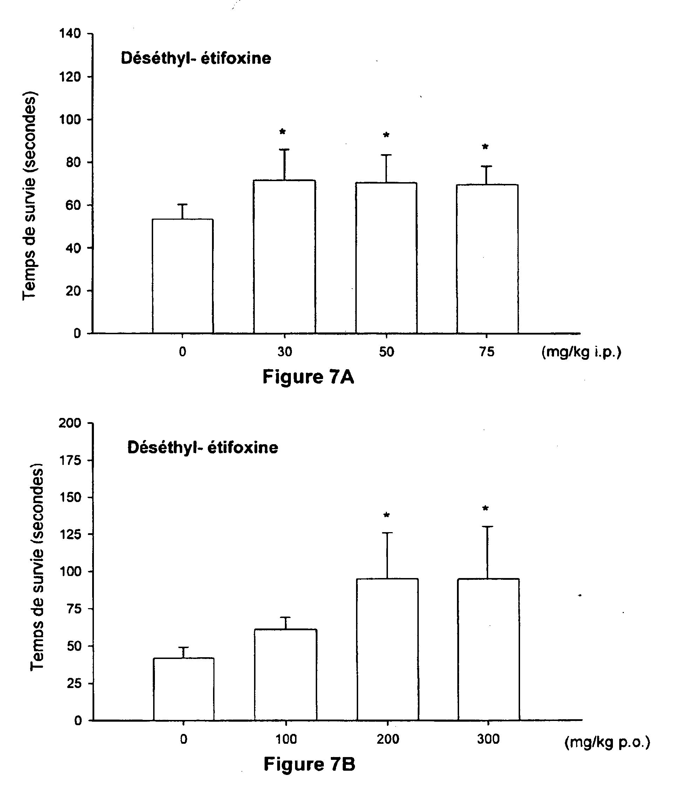

   Les Figures 7A et 7B représentent les effets de concentrations croissantes de déséthyl-étifoxine (en abscisses, mg/kg) administrées par voie intrapéritonéale (Figure 7A) ou par voie orale (Figure 7B) sur le temps de survie (en ordonnées, en secondes, moyenne ± écart standard à la moyenne) de souris soumises à une hypoxie hypobare induite par une diminution de la pression atmosphérique à 160 mmHg (10 à 20 animaux par groupe). Le signe étoile (*) représente p<0,05 par rapport aux témoins respectifs, dans le test statistique ANOVA.
**Figure 8A et Figure 8B**

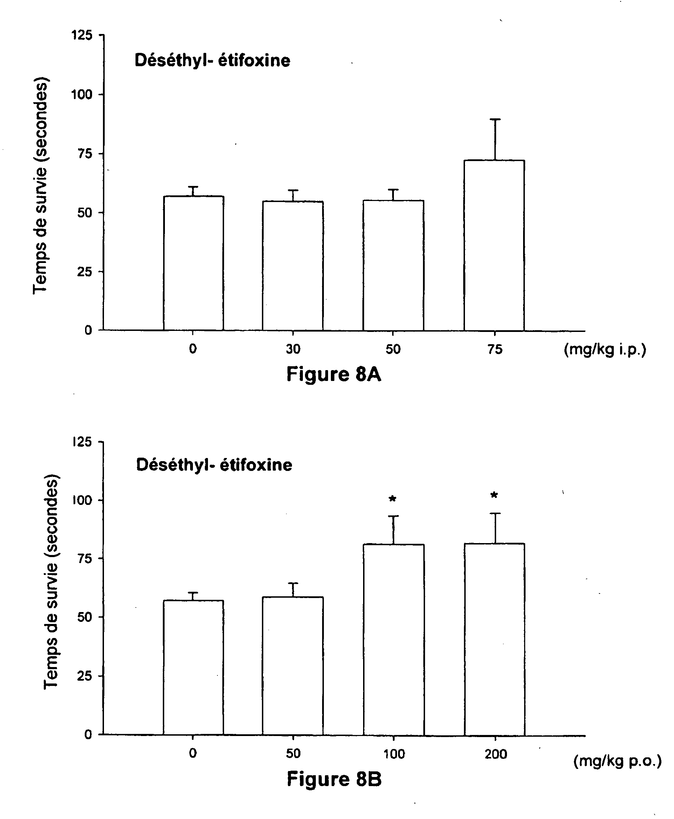

   Les Figures 8A et 8B représentent les effets de concentrations croissantes de déséthyl-étifoxine (en abscisses, mg/kg) administrées par voie intrapéritonéale (Figure 8A) ou par voie orale (Figure 8B) sur le temps de survie (en ordonnées, en secondes, moyenne ± écart standard à la moyenne) de rats soumis à une hypoxie histotoxique induite par une administration intraveineuse de 4 mg/kg de cyanure de potassium (10 animaux par groupe). Le signe étoile (*) représente p<0,05 par rapport aux témoins respectifs, dans le test statistique ANOVA.
**Figure 9**
   La Figure 9 représente l'impact des traitements de la maladie de Parkinson ; adapté de Fénélon (2005) Rev. Prat. 714-716.

### EXEMPLES

### EXEMPLE 1

### Evaluation des effets neuroprotecteurs de l'étifoxine dans des cultures mélangées de neurones corticaux et d'astrocytes de rats en présence d'un excès de glutamate

Le principe de l'étude a consisté à évaluer la survie des neurones corticaux en coculture avec des astrocytes après application d'un neurotoxique, le glutamate en excès.

En effet, le glutamate est responsable d'une excitotoxicité (activation excessive de récepteurs neuronaux) impliquée dans la mort neuronale consécutive à de nombreux troubles, dont les accidents vasculaires cérébraux, les crises d'épilepsie ou certaines affections neurodégénératives, telles que la maladie de Huntington ou la sclérose latérale amyotrophique.

La survie neuronale est quantifiée par l'importance de la densité du réseau de prolongements neuritiques marqué par un anti-corps anti-neurofilaments en absence ou en présence de glutamate.

### 1. Matériels et méthodes

### 1.1. Cultures cellulaires

- **Type cellulaire :**: Primo culture de neurones corticaux d'embryons de rat de 14 jours et primo culture d'astrocytes corticaux de rats nouveaux nés.
- **Milieu de culture :**: DMEM-Ham F12 (Invitrogen 21331020)
B27 2% (invitrogen 17504044)
L-glutamine 2mM (Invitrogen 25030024)
Pénicilline 50 Ul/ml - Streptomycine 50 µg/ml (Invitrogen 15070063)
Nerf Growth Factor 10 ng/ml (NGF, Invitrogen 13290.010)
- **Milieu de survie :**: MEM (Invitrogen 21090022)
L-glutamine 2Mm (Invitrogen 25030024)
Pénicilline 50 Ul/ml - Streptomycine 50 µg/ml (Invitrogen 15070063)
Supplément N2 (Invitrogen 17502-048)

### 1.2 Cytotoxicité préalable

| | |
|---|---|
| Plaques : | 96 puits |
| Temps de culture : | 8 jours |
| Cellules/puits : | neurones corticaux + astrocytes |
| Gamme de produit : | à partir de 100 µM puis raison 3 |
| Replicata : | 3 |
| Contact cellules/produit : | 48 heures |
| Paramètres d'évaluation : | observation microscopique et hydrolyse au MTT. |
| MTT : | Bromure de 3-(4,5-dimethylthiozol-2-yl)-2, 5-diphényltétrazolium. |

### 1.3 Traitement et analyse du réseau des prolongements marqués avec un anticorps anti-neurofilaments

Les neurones corticaux ont été prélevés sur des cortex d'embryons de rat de 14 jours et cultivés en milieu de culture en plaque 96 puits dans une étuve à 37° C et 5% CO₂ saturée en humidité. Après 2 jours de culture, des astrocytes issus de cortex de rats nouveaux nés ont été ensemencés dans les puits (selon un ratio de 1 astrocyte pour 4 neurones). Après 10 jours de culture, les neurones matures synthétisent les neurofilaments (protéine de structure spécifique des neurones matures) ; de plus ces neurones expriment à leur surface des récepteurs fonctionnels au glutamate et peuvent donc être intoxiqués.

Au 12^{ème} jour de culture, le milieu a été remplacé par du milieu de survie avec ou sans étifoxine à 3 concentrations et les neurones ont été intoxiqués ou non par du glutamate (Sigma G1501) à 2 mM et 10 mM. Chaque condition de culture a été réalisée dans 4 puits. Les cultures ont été incubées pendant 2 temps (6 et 24 heures).

Un contrôle positif a été réalisé en milieu de culture de survie contenant du Nerve Growth Factor (NGF ; 10 ng/ml) et du Fibroblast Growth Factor basic (FGFb ; 5 ng/ml) et les cellules ont été intoxiquées dans les mêmes conditions.

Au terme des deux temps d'incubation (6 et 24 h), les tapis cellulaires ont été fixés et marqués par un anticorps monoclonal anti-neurofilaments 68 et 200 kD (DAKO M0762) puis révélés par un conjugué anticorps de chèvre anti-immunoglobuline de souris-Alexia fluor 488 (Interchim A-11029). Des contrôles sans anticorps primaire ont été réalisés. Tous les contrôles étaient négatifs (pas de marquage non spécifique). Après lavages extensifs en PBS, les préparations ont été observées en épi-fluorescence (microscope Nikon Diaphot 300).

Pour chaque puits de culture (4 puits par condition expérimentale), 2 images ont été réalisées à l'aide d'une caméra Nikon DXM 1 200F pilotée par un logiciel LUCIA 6.0.

Toutes les images ont été effectuées dans les mêmes conditions et avec des réglages de la caméra identiques.

Des analyses de la densité des réseaux de prolongements marqués avec l'anticorps anti-neurofilaments ont été réalisées à l'aide du logiciel LUCIA 6.0. Un premier traitement des images a permis d'augmenter l'intensité du marquage spécifique des prolongements. Les zones positives au marquage ont été binarisées et le pourcentage de marquage par image a été analysé (surface marquée/surface totale analysée).

### 1.4. Produit étudié

L'étifoxine (chlorhydrate de 6-chloro-2-éthylamino-4-méthyl-4-phényl-4H-[3,1]benzoxazine) est mise en solution à la concentration de 100 mM dans le DMSO puis diluée successivement dans le milieu de culture (concentration de DMSO≤0,1 %, v/v).

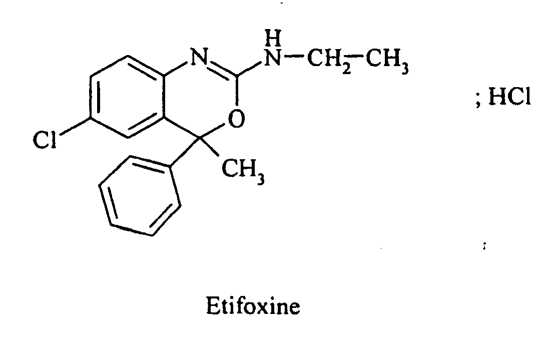

### 1.5. Expression et analyse statistique des résultats

Les résultats (valeurs moyennes ± erreur standard à la moyenne (ESM), sont exprimés en pourcentage de marquage par champs de microscope (n = 8 mesures/conditions de culture). L'analyse de variance à 2 facteurs (Etifoxine et glutamate), suivie du test de comparaisons multiples de Student Newman-Keuls a été utilisée pour la comparaison statistique des résultats.

Les résultats obtenus avec le contrôle positif (FGF) ont été comparés aux témoins à l'aide du test t de Student ou de Mann et Whitney selon les cas. Le seuil de significativité était fixé à p<0.05 (logiciel SigmaStat - V3.1, SPSS inc).

### 2. Résultats

### 2.1 Cytotoxicité de l'étifoxine

Une première expérience a été réalisée (test classique utilisé pour mesurer le taux de survie cellulaire) dans le but d'étudier la cytotoxicité de l'étifoxine vis-à-vis des neurones corticaux en présence d'astrocytes en culture et de déterminer ainsi la concentration maximale non toxique d'étifoxine.

L'analyse visuelle réalisée au microscope après 48 h d'incubation montre une mortalité neuronale à la concentration de 33 et 100 µM d'étifoxine tandis que la mortalité des astrocytes est observée à la concentration de 100 µM. Les concentrations d'étifoxine retenues pour l'étude proprement dite sont 1, 3 et 10 µM.

### 2.2 Effets de l'étifoxine sur la densité de neurofilaments en présence de glutamate après 6 h d'incubation (Figure 1)

Dans le milieu de culture contrôle (sans glutamate), l'étifoxine accroît de manière dose dépendante la densité de neurofilaments marqués avec un effet statistiquement significatif à la dose de 10 µM.

Le glutamate aux concentrations de 2 et 10 mM réduit de manière statistiquement significative la densité de neurofilaments. L'étifoxine aux concentrations de 3 et 10 µM, ainsi que le FGF s'opposent de manière statistiquement significative à la diminution de la densité de neurofilaments induite par le glutamate.

### 2.3 Effets l'étifoxine sur la densité de neurofilaments en présence de glutamate après 24 h (Figure 2)

Dans le milieu de culture contrôle, comme précédemment, l'étifoxine accroît de manière statistiquement significative la densité de neurofilaments à la dose de 10 µM. Les concentrations de 1 et 3 µM sont sans effet.

Le glutamate, aux concentrations de 2 et 10 mM réduit de manière importante le marquage des neurofilaments qui est proche de 0.

L'étifoxine à la concentration de 10 µM et le FGF s'opposent de manière statistiquement significative aux effets de 2 mM de glutamate.

Les résultats révèlent d'une part que l'étifoxine possède un effet sur la croissance neuronale ou effet neurotrophique comme le montre l'augmentation de la densité des réseaux de neurofilaments dans la culture contrôle après 6 et 24 h d'incubation.

D'autre part, ils mettent aussi en évidence un effet neuroprotecteur de l'étifoxine après intoxication par le glutamate. En effet, l'étifoxine, comme les facteurs de croissance, s'oppose à la réduction des réseaux de neurofilaments induite par un excès de glutamate.

### EXEMPLE 2

### Evaluation des effets neuroprotecteurs de la déséthyl-étifoxine dans des cultures mélangées de neurones corticaux et d'astrocytes de rats en présence d'un excès de glutamate

La méthodologie de **l'Exemple 1** a été appliquée à un métabolite actif de l'étifoxine, à savoir la déséthyl-étifoxine (2-amino-6-chloro-4-méthyl-4-phényl-4H-[3,1]benzoxazine), qui a été mise en solution à la concentration de 100 mM dans le DMSO puis diluée successivement dans le milieu de culture (concentration de DMSO≤0,1 %, v/v).

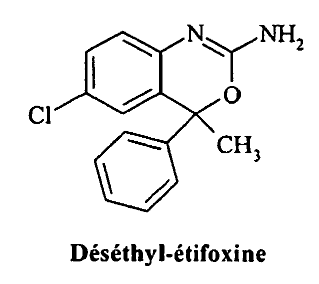

### Cytotoxicité de la déséthyl-étifoxine

L'analyse visuelle réalisée au microscope après 48 h d'incubation montre une mortalité neuronale à la concentration de 33 et 100 µM de déséthyl-étifoxine tandis que la mortalité des astrocytes est observée à la concentration de 100 µM. Les concentrations de déséthyl-étifoxine retenues pour l'étude proprement dite ont été 1, 3 et 10 µM.

### Effets de la déséthyl-étifoxine sur la densité de neurofilaments en présence de glutamate après 6h d'incubation (Figure 3)

Dans le milieu de culture contrôle (sans glutamate), la déséthyl-étifoxine accroît de manière statistiquement significative la densité de neurofilaments marqués à la dose de 10 µM.

Le glutamate à la concentration de 2 mM réduit de manière statistiquement significative la densité de neurofilaments. Seule la concentration de 10 µM de déséthyl-étifoxine s'oppose à l'effet du glutamate. Par contre, la déséthyl-étifoxine aux concentrations de 1, 3 et 10 µM, s'oppose de manière statistiquement significative à la diminution de la densité de neurofilaments marqués induite par 10 µM de glutamate.

### EXEMPLE 3

### Effet protecteur de l'étifoxine dans 3 modèles d'hypoxie chez la souris et le rat

De nombreuses situations pathologiques, telles que les crises d'épilepsie ou les accidents vasculaires cérébraux, se traduisent par une hypoxie, locale ou généralisée, des tissus nerveux, qui conduit à leur destruction, partielle ou totale, notamment par mort neuronale. Chez l'animal placé en condition d'hypoxie sévère, la destruction du tissu nerveux conduit rapidement à la mort de l'animal. Les effets de l'étifoxine sur la durée de survie de souris ou de rats placés en situation d'hypoxie a donc été étudiée afin d'évaluer son efficacité neuroprotectrice.

### 1. Matériel et méthodes

### 1.1 Hypoxie hypobare chez la souris

### Animaux

Des souris mâles NMRI de chez Janvier, de poids compris entre 25 et 30 grammes sont utilisées après une acclimatation d'au moins 7 jours dans l'animalerie (t° = 22 ± 2° C ; hygrométrie 50 ± 20 % ; nourriture UAR "A04" SAFE (Augy, France) et eau du robinet *ad libitum* ; cycle nycthéméral de 12 heures (lumière de 7 à 19 heures).

### Protocole

Les souris non à jeun, sont réparties au hasard par lot de 10.

Le protocole de Nakaniski et al. (1973) Life Sciences, 13, 467-474 est adapté pour réaliser le modèle.

Chaque souris est placée dans un dessiccateur (enceinte hermétique) dans lequel on diminue la pression atmosphérique de 760 à 160 mmHg à l'aide d'une trompe à eau. Cette dépression est effectuée en une minute et provoque la mort chez les animaux témoins en 1 minute environ.

On note alors le temps de survie de l'animal qui correspond à la différence entre le temps d'apparition de la mort de la souris, appréciée par l'arrêt respiratoire, moins le temps nécessaire à l'induction de l'hypoxie.

Les produits à étudier sont administrés par voie intrapéritonéale (i.p.) (0,1 ml/10 g), 30 minutes avant l'induction de l'hypoxie hypobare, ou par voie orale (0,1 ml/10 g p.o.) 1 heure avant l'hypoxie.

### 1.2 Hypoxie au KCN (i.p) chez la souris

### Animaux

Des souris mâles CD1 de chez Charles River, de poids compris entre 20 et 25 grammes sont utilisées après une acclimatation d'au moins 7 jours dans l'animalerie (t° = 22 ± 2° C ; hygrométrie 50 ± 20 % ; nourriture UAR "A04" ; cycle nycthéméral de 12 heures (lumière de 7 à 19 heures).

### Protocole

Les souris non à jeun, sont réparties au hasard par lot de 10.

Chaque souris reçoit par voie intrapéritonéale (i.p) (0,1 ml/10 g) une solution extemporanée de cyanure de potassium (KCN) dans du NaCl à 0,9 % (1,5 mg/ml). Cela correspond à une dose de 15 mg/kg de KCN qui provoque la mort chez les animaux témoins en quelques minutes.

On note le temps d'apparition de la mort pour chaque souris, appréciée par l'arrêt cardiaque.

Les produits à étudier sont administrés par voie intrapéritonéale (i.p.) (0,1 ml/10 g), 30 minutes avant l'injection de KCN, ou par voie orale (0,1 ml/10 g p.o.) 1 heure avant le KCN.

### 1.3 Hypoxie au KCN (i.v.) chez le rat

### Animaux

Des rats mâles Wistar de chez Janvier, de poids compris entre 180 et 200 grammes sont utilisées après une acclimatation d'au moins 7 jours dans l'animalerie (t° = 22 ± 2° C ; hygrométrie 50 ± 20 % ; nourriture UAR "A04" SAFE (Augy, France) ; cycle nycthéméral de 12 heures (lumière de 7 à 19 heures).

### Protocole

Les rats non à jeun, sont répartis au hasard par lot de 10.

Le protocole est adapté d'après Lamar et al. (1988) Drug Develop. Res., 14, 297-304.

Chaque rat reçoit par voie intraveineuse (i.v.) (0,1 ml/100 g) une solution extemporanée de cyanure de potassium (KCN) dans du NaCl à 0,9 % (4 mg/ml). Cela correspond à une dose de 4 mg/kg de KCN qui provoque la mort chez les animaux témoins en quelques minutes.

On note le temps d'apparition de la mort pour chaque rat, appréciée par l'arrêt cardiaque.

Les produits à étudier sont administrés par voie intrapéritonéale (i.p.) (0,5 ml/100 g), 30 minutes avant l'injection de KCN, ou par voie orale (0,5 ml/100g p.o.) 1 heure avant le KCN.

### 1.4 Produits

Le chlorhydrate d'étifoxine est mis en solution dans du Tween 80 à 1 %. Le cyanure de potassium (Merck) est solubilisé dans du NaCl à 0,9 %.

### 1.5 Statistiques

Le test statistique utilisé est une analyse de variance à un facteur pour déterminer les groupes traités qui diffèrent du groupe témoin recevant le véhicule, au seuil de 5 %.

### 2. Résultats

### 2.1 Hypoxie hypobare chez la souris

L'induction de la dépression pour obtenir une hypoxie hypobare entraîne la mort en 40 à 60 secondes chez les souris sans traitement ou recevant le liquide véhicule.

L'étifoxine augmente de manière significative le délai d'apparition de la mort à partir de 30 mg/kg i.p ou p.o **(Tableau 1 et** **Figures 4A-4B****).**

**Tableau 1 : Effets de l'étifoxine sur le temps de survie dans l'hypoxie hypobare induite par une diminution de la pression atmosphérique à 160 mmHg chez la souris (n nombre d'animaux, m moyenne, esm écart standard de la moyenne).**

| **Voies** | **Doses (mg/kg)** | **n** | **Temps de survie en secondes** **(m ± esm)** | **Pourcentages de variation** | **Test statistique** **ANOVA** |
|---|---|---|---|---|---|
| i.p. | 0 | 20 | 57,5 ± 2,0 | | |
| | 3 | 10 | 64,5 ± 3,4 | + 12 | ns |
| | 10 | 19 | 66,1 ± 3,0 | + 15 | ns |
| | 30 | 19 | 81,8 ± 4,6 | + 42 | p < 0,05 |
| | 50 | 10 | 138,5 ± 23,3 | + 141 | p < 0,05 |
| | 75 | 10 | 132,0 ± 21,6 | + 130 | p < 0,05 |
| p.o. | 0 | 20 | 55,5 ± 2,3 | | |
| | 30 | 10 | 81,5 ± 4,5 | + 47 | p < 0,05 |
| | 100 | 20 | 95,3 ± 5,6 | + 72 | p < 0,05 |
| | 200 | 10 | 99,5 ± 2,7 | + 79 | p < 0,05 |
| | 300 | 20 | 115,5 ± 8,6 | + 108 | p < 0,05 |

### 2.2 Hypoxie au KCN (i.p.) chez la souris

L'administration de 15 mg/kg i.p de KCN entraîne la mort en 90 à 120 secondes chez les souris sans traitement ou recevant le véhicule liquide.

L'étifoxine augmente de manière significative le délai d'apparition de la mort à partir de 50 mg/kg i.p, mais ne modifie pas significativement le temps de survie par voie orale **(Tableau 2 et** **Figures 5A-5B****).**

**Tableau 2 : Effets de l'étifoxine sur le temps de survie dans l'hypoxie histotoxique induite par le cyanure de potassium (15 mg/kg i.p.) chez la souris (n nombre d'animaux, m moyenne, esm écart standard de la moyenne).**

| **Voies** | **Doses** | **n** | **Temps de survie en secondes** **(m ± esm)** | **Pourcentages de variation** | **Test statistique** **ANOVA** |
|---|---|---|---|---|---|
| i.p. | 0 | 19 | 106 ± 9 | | |
| | 3 | 19 | 122 ± 7 | + 15 | ns |
| | 10 | 20 | 113 ± 5 | + 7 | ns |
| | 30 | 18 | 129 ± 11 | + 22 | ns |
| i.p. | 0 | 19 | 91 ± 4 | | |
| | 30 | 16 | 108 ± 4 | + 19 | ns |
| | 50 | 18 | 124 ± 8 | + 36 | p < 0,05 |
| | 75 | 17 | 133 ± 6 | + 46 | p < 0,05 |
| p.o. | 0 | 9 | 108 ± 11 | | |
| | 100 | 9 | 94 ± 6 | - 13 | ns |
| | 200 | 10 | 123 ± 12 | + 14 | ns |
| | 300 | 10 | 102 ± 7 | - 6 | ns |

### 2.3 Hypoxie au KCN (i.v) chez le rat

L'administration de 4 mg/kg i.v de KCN entraîne la mort en 50 à 60 secondes chez les rats sans traitement ou recevant le véhicule liquide.

L'étifoxine augmente de manière significative le délai d'apparition de la mort à partir de 50 mg/kg i.p et 200 mg/kg p.o **Tableau 3** et **Figures 6A-6B****).**

**Tableau 3 : Effets de l'étifoxine sur le temps de survie dans l'hypoxie histotoxique induite par le cyanure de potassium (4 mg/kg i.v.) chez le rat (n nombre d'animaux, m moyenne, esm écart standard de la moyenne).**

| **Voies** | **Doses** | **n** | **Temps de survie en secondes** **(m ± esm)** | **Pourcentages de variation** | **Test statistique** **ANOVA** |
|---|---|---|---|---|---|
| i.p. | 0 | 10 | 56,6 ± 2,6 | | |
| | 30 | 10 | 60,3 ± 2,0 | + 7 | ns |
| | 50 | 10 | 107,3 ± 6,1 | + 90 | p < 0,05 |
| | 75 | 10 | 144,3 ± 11,5 | + 155 | p < 0,05 |
| p.o. | 0 | 9 | 55,4 ± 1,1 | | |
| | 50 | 10 | 59,5 ± 2,1 | + 7 | ns |
| | 100 | 10 | 60,1 ± 1,2 | + 8 | ns |
| | 200 | 10 | 63,9 ± 2,0 | + 15 | p < 0,05 |

### EXEMPLE 4

La méthodologie de l'**Exemple 3** a été appliquée à la déséthyl-étifoxine qui a été mise en solution dans du Tween 80 à 1 %.

### 1 Hypoxie hypobare chez la souris

La déséthyl-étifoxine montre une activité anti-hypoxique à partir de 30 mg/kg i.p.. Par voie orale, elle entraîne une augmentation significative de la durée de survie à partir de 200 mg/kg **(Tableau 4 et** **Figures 7A-7B****).**

**Tableau 4 : Effets de la déséthyl-étifoxine sur le temps de survie dans l'hypoxie hypobare induite par une diminution de la pression atmosphérique à 160 mmHg chez la souris (n nombre d'animaux, m moyenne, esm écart standard de la moyenne).**

| **Voies** | **Doses** | **n** | **Temps de survie en secondes** **(m ± esm)** | **Pourcentages de variation** | **Test statistique** **ANOVA** |
|---|---|---|---|---|---|
| i.p. | 0 | 10 | 53,5 ± 2,2 | | |
| | 30 | 9 | 71,7 ± 4,8 | + 34 | p < 0,05 |
| | 50 | 9 | 70,7 ± 4,3 | + 32 | p < 0,05 |
| | 75 | 10 | 69,8 ± 2,7 | + 30 | p < 0,05 |
| p.o. | 0 | 10 | 42,0 ± 2,3 | | |
| | 100 | 10 | 61,0 ± 2,6 | + 45 | ns |
| | 200 | 10 | 94,9 ± 9,9 | + 126 | p < 0,05 |
| | 300 | 10 | 94,8 ± 11,2 | + 126 | p < 0,05 |

### 2 Hypoxie au KCN (i.p.) chez le rat

Dans ce test, la déséthyl-étifoxine augmente le temps de survie de manière non significative à 75 mg/kg i.p. en raison d'une forte variabilité. Par voie orale, elle montre une activité anti-hypoxique à partir de 100 mg/kg p.o **(Tableau 5 et** **Figures 8A-8B****).**

**Tableau 5 : Effets de la déséthyl-étifoxine sur le temps de survie dans l'hypoxie histotoxique induite par le cyanure de potassium (4 mg/kg i.p.) chez le rat (n nombre d'animaux, m moyenne, esm écart standard de la moyenne).**

| **Voies** | **Doses** | **n** | **Temps de survie en secondes** **(m ± esm)** | **Pourcentages de variation** | **Test statistique** **ANOVA** |
|---|---|---|---|---|---|
| i.p. | 0 | 10 | 57,1 ± 1,2 | | |
| | 30 | 10 | 55,0 ± 1,5 | - 4 | ns |
| | 50 | 10 | 55,6 ± 1,5 | - 3 | ns |
| | 75 | 10 | 72,7 ± 5,5 | + 27 | ns |
| p.o. | 0 | 10 | 57,3 ± 1,0 | | |
| | 50 | 10 | 58,8 ± 1,9 | + 3 | ns |
| | 100 | 10 | 81,3 ± 3,8 | + 42 | p < 0,05 |
| | 200 | 10 | 81,7 ± 4,1 | + 43 | p < 0,05 |

## Revendications

1. Utilisation d'au moins un composé de formule (I) suivante : 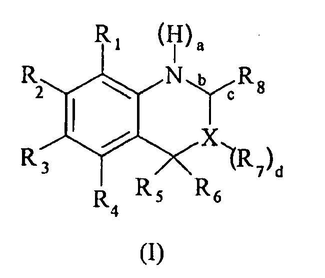 dans laquelle :
- a représente 0 ou 1 ;
- b représente une simple liaison ou une double liaison ;
- c représente une simple liaison ou une double liaison ;
- d représente 0 ou 1 ;
- X représente un atome d'oxygène ou d'azote, sous réserve que lorsque X représente un atome d'oxygène alors d vaut 0 et que lorsque X représente un atome d'azote alors d vaut 1 ;
- R₁, R₂, R₃, et R₄, identiques ou différents, représentent un atome d'hydrogène, un atome d'halogène, notamment choisi parmi F, Cl, Br, ou I, un groupe hydroxyle, ou un groupe alkoxyle de 1 ou 2 atomes de carbone ;
- R₅ et R₆, identiques ou différents, représentent un atome d'hydrogène, un groupe alkyle ou cycloalkyle de 1 à 6 atomes de carbone, ou un groupe aryle de 6 atomes de carbone dont le noyau aromatique est éventuellement substitué par un ou plusieurs atomes d'halogène ou un ou plusieurs groupes hydroxyle, alkoxyle de 1 ou 2 atomes de carbone, trifluorométhyle ou nitro ;
- R₇ représente un atome d'hydrogène, un groupe hydroxyle, ou un groupe alkyle ou hydroxyalkyle de 1 à 3 atomes de carbone ;
- R₈ représente un atome d'oxygène ou un groupe -NR₉R₁₀, R₉ et R₁₀, identiques ou différents, représentant un atome d'hydrogène, un groupe hydroxyle, ou un groupe alkyle ou hydroxyalkyle de 1 à 3 atomes de carbone, sous réserve que lorsque R₈ représente un atome d'oxygène alors a vaut 1, b représente une simple liaison et c représente un double liaison et que lorsque R₈ représente un groupe -NR₉R₁₀ alors a vaut 0, b représente une double liaison et c représente une simple liaison ;
ou de ses sels pharmaceutiquement acceptables,
pour la préparation d'un médicament à activité neuroprotectrice destiné au traitement des détériorations neuronales.

2. Utilisation selon la revendication 1, pour la préparation d'un médicament ayant une activité de régénération des cellules du système nerveux central destiné au traitement des détériorations neuronales.

3. Utilisation selon la revendication 1 ou 2, dans laquelle les détériorations neuronales sont liées aux pathologies choisies dans la liste comprenant :
- l'épilepsie,
- les accidents vasculaires cérébraux, ischémiques ou hémorragiques,
- les maladies neurodégénératives, telles que la maladie de Charcot-Marie-Tooth ou la Maladie de Friedreich,
- les phacomatoses, notamment les neurofibromatoses,
- les neuropathies, telles que les neuropathies carentielles, notamment alcooliques, les neuropathies toxiques ou médicamenteuses, notamment à la vincristine, les neuropathies liées à une affection métabolique, telle que le diabète, les neuropathies liées à un processus inflammatoire, notamment le syndrome de Guillain-Barré, les neuropathies infectieuses, notamment le zona, et les neuropathies radiques,
- la polynévrite paranéoplasique,
- la sclérose en plaque,
- la sclérose latérale amyotrophique,
- la schizophrénie,
- la dépression,
- les tumeurs cérébrales,
- la maladie de Parkinson, et
- les démences, telles que la maladie d'Alzheimer, la maladie de Pick ou la démence vasculaire.

4. Utilisation selon l'une des revendications 1 à 3, d'un composé de formule (II) suivante : 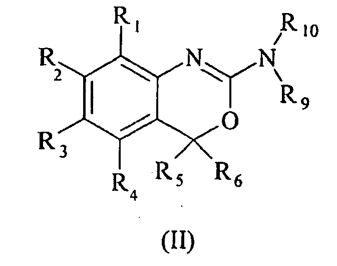 dans laquelle R₁, R₂, R₃, R₄, R₅, R₆, R₉ et R₁₀ sont tels que définis ci-dessus.

5. Utilisation selon l'une des revendications 1 à 4, des composés de formules (III) et (IV) suivantes :

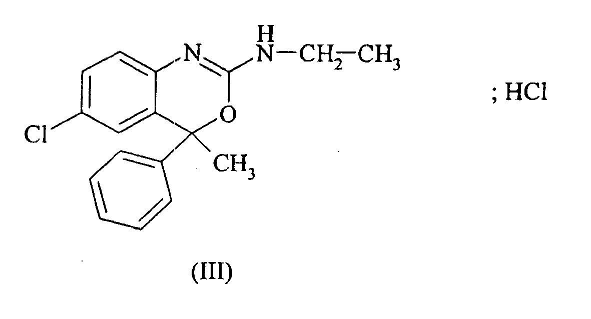

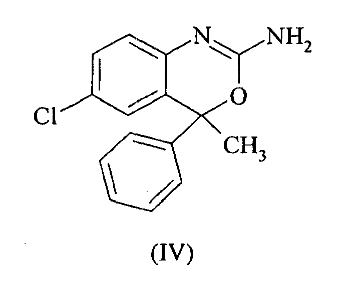

6. Utilisation selon l'une des revendications 1 à 3, d'un composé de formule (V) suivante : 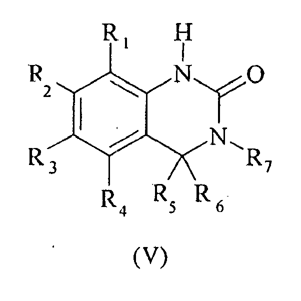 dans laquelle R₁, R₂, R₃, R₅, R₆, et R₇ sont tels que définis dans la revendication 1.

7. Utilisation selon la revendication 1, 2, 3 ou 6, de composés de formules (VI) et (VII) suivantes :

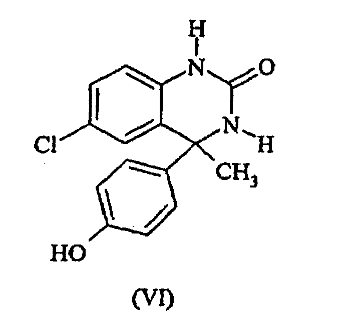

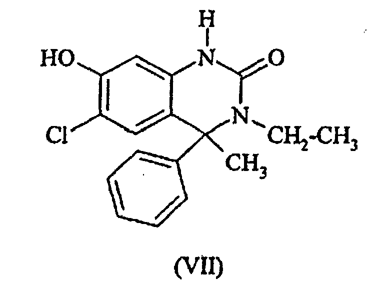

8. Utilisation selon l'une des revendications 1 à 7, dans laquelle le médicament convient pour l'administration à un individu en ayant besoin d'une dose unitaire de 50 mg à 1500 mg, notamment de 150 à 200 mg du composé tel que défini dans l'une des revendications 1 et 4 à 7.

9. Utilisation selon l'une des revendications 1 à 8, dans laquelle le médicament convient pour l'administration à un individu en ayant besoin d'une dose de 50 mg/j à 1500 mg/j, notamment de 150 mg/j à 200 mg/j, du composé tel que défini dans l'une des revendications 1 et 4 à 7.

10. Utilisation selon l'une des revendications 1 à 9, dans laquelle le médicament convient pour une administration par voie orale.

11. Utilisation selon l'une des revendications 1 à 10, dans laquelle le médicament se présente sous la forme d'une poudre, de cachets, de gélules ou de sachets.

12. Utilisation selon la revendication 1, dans laquelle le composé est l'étifoxine et le médicament exerce un effet neurotrophique.

13. Utilisation selon l'une des revendications 1 à 11, dans laquelle le composé tel que défini dans l'une des revendications 1 et 4 à 7 est associé à au moins un composé additionnel destiné à la prévention ou au traitement des pathologies définies dans la revendication 3.

14. Produits contenant :
• au moins un composé de formule (I) tel que défini dans l'une des revendications 1 et 4 à 7 ou ses sels pharmaceutiquement acceptables, et
• au moins un composé additionnel destiné à la prévention ou au traitement des pathologies choisies dans la liste comprenant :
- l'épilepsie,
- les accidents vasculaires cérébraux, ischémiques ou hémorragiques,
- les maladies neurodégénératives, telles que la maladie de Charcat-Marie-Tooth ou la Maladie de Friedreich,
- les phacomatoses, notamment les neurofibromatoses,
- les neuropathies, telles que les neuropathies carentielles, notamment alcooliques, les neuropathies toxiques ou médicamenteuses, notamment à la vincristine, les neuropathies liées à une affection métabolique, telle que le diabète, les neuropathies liées à un processus inflammatoire, notamment le syndrome de Guillain-Barré, les neuropathies infectieuses, notamment le zona, et les neuropathies radiques,
- la polynévrite paranéoplasique,
- la sclérose en plaque,
- la sclérose latérale amyotrophique,
- la schizophrénie,
- la dépression,
- les tumeurs cérébrales,
- la maladie de Parkinson, et
- les démences, telles que la maladie d'Alzheimer, la maladie de Pick ou la démence vasculaire,
comme produit de combinaison pour une utilisation séparée, simultanée ou étalée dans le temps pour le traitement des détériorations neuronales liées à ces pathologies.

15. Composé de formule (I) tel que défini dans l'une des revendications 1 et 4 à 7, ou un de ses sels pharmaceutiquement acceptables, pour son utilisation à titre de médicament à activité neuroprotectrice dans le traitement des détériorations neuronales.

16. Composé de formule (I), ou un de ses sels pharmaceutiquement acceptables, pour son utilisation selon la revendication 15, à titre de médicament à activité de régénération des cellules du système nerveux central dans le traitement des détériorations neuronales.

17. Composé de formule (I), ou un de ses sels pharmaceutiquement acceptables, pour son utilisation selon la revendication 15 ou 16, dans lequel les détériorations neuronales sont liées aux pathologies choisies dans la liste comprenant :
- l'épilepsie,
- les accidents vasculaires cérébraux, ischémiques ou hémorragiques,
- les maladies neurodégénératives, telles que la maladie de Charcot-Marie-Tooth ou la Maladie de Friedreich,
- les phacomatoses, notamment les neurofibromatoses,
- les neuropathies, telles que les neuropathies carentielles, notamment alcooliques, les neuropathies toxiques ou médicamenteuses, notamment à la vincristine, les neuropathies liées à une affection métabolique, telle que le diabète, les neuropathies liées à un processus inflammatoire, notamment le syndrome de Guillain-Barré, les neuropathies infectieuses, notamment le zona, et les neuropathies radiques,
- la polynévrite paranéoplasique,
- la sclérose en plaque,
- la sclérose latérale amyotrophique,
- la schizophrénie,
- la dépression,
- les tumeurs cérébrales,
- la maladie de Parkinson, et
- les démences, telles que la maladie d'Alzheimer, la maladie de Pick ou la démence vasculaire.

18. Composé de formule (I), ou un de ses sels pharmaceutiquement acceptables, pour son utilisation selon l'une des revendications 15 à 17, dans laquelle le composé tel que défini dans l'une des revendications 1 et 4 à 7 est associé à au moins un composé additionnel destiné à la prévention ou au traitement des pathologies définies dans la revendication 17.

19. Composé de formule (I), ou un de ses sels pharmaceutiquement acceptables pour son utilisation selon la revendication 15, dans laquelle le composé est l'étifoxine et il exerce un effet neurotrophique.

## Patentansprüche

1. Verwendung mindestens einer Verbindung der folgenden Formel (I) : 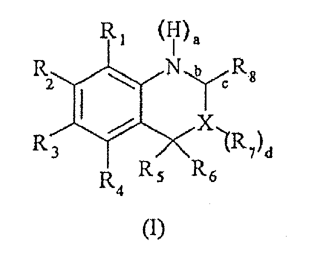 worin:
- a für 0 oder 1 steht;
- b für eine Einfachbindung oder eine Doppelbindung steht;
- c für eine Einfachbindung oder eine Doppelbindung steht;
- d für 0 oder 1 steht;
- X für ein Sauerstoffatom oder ein Stickstoffatom steht, mit der Maßgabe, dass, wenn X für ein Sauerstoffatom steht, d 0 ist und dass, wenn X für ein Stickstoffatom steht, d 1 ist;
- R₁, R₂, R₃ und R₄ gleich oder verschieden sind und für ein Wasserstoffatom, ein Halogenatom, insbesondere ausgewählt aus F, Cl, Br oder I, eine Hydroxylgruppe oder eine Alkoxygruppe mit 1 oder 2 Kohlenstoffatomen stehen;
- R₅ und R₆ gleich oder verschieden sind und für ein Wasserstoffatom, eine Alkyl- oder Cycloalkylgruppe mit 1 bis 6 Kohlenstoffatomen oder eine Arylgruppe mit 6 Kohlenstoffatomen, wobei der aromatische Kern gegebenenfalls substituiert ist mit einem oder mehreren Halogenatom(en) oder einer oder mehreren Hydroxylgruppe(n), Alkoxygruppen mit 1 oder 2 Kohlenstoffatom(en), Trifluormethylen oder Nitro, stehen;
- R₇ für ein Wasserstoffatom, eine Hydroxylgruppe oder eine Alkyl- oder Hydroxyalkylgruppe mit 1 bis 3 Kohlenstoffatomen steht;
- R₈ für ein Sauerstoffatom oder eine -NR₉R₁₀-Gruppe steht, wobei R₉ und R₁₀ gleich oder verschieden sind und für ein Wasserstoffatom, eine Hydroxylgruppe oder eine Alkylgruppe oder Hydroxyalkylgruppe mit 1 bis 3 Kohlenstoffatomen stehen mit der Maßgabe, dass, wenn R₈ für ein Sauerstoffatom steht, a 1 ist, b für eine Einfachbindung steht und c für eine Doppelbindung steht und dass, wenn R₈ für eine -NR₉R₁₀-Gruppe steht, a 0 ist, b für eine Doppelbindung steht und c für eine Einfachbindung steht;
oder ihrer pharmazeutisch annehmbaren Salze,
zur Herstellung eines Medikaments mit neuroprotektiver Wirkung, das zur Behandlung von neuronalen Schädigungen bestimmt ist.

2. Verwendung nach Anspruch 1 zur Herstellung eines Medikaments mit regenerierender Wirkung auf die Zellen des Zentralnervensystems, das zur Behandlung von neuronalen Schädigungen bestimmt ist.

3. Verwendung nach Anspruch 1 oder 2, wobei die neuronalen Schädigungen bedingt sind durch Pathologien, ausgewählt aus der Liste, umfassend:
- Epilepsie,
- zerebrovaskulären, ischämischen oder hämorrhagischen Insult,
- neurodegenerative Krankheiten, wie die Charcot-Marie-Tooth-Krankheit oder die Friedreich-Krankheit,
- Phakomatosen, insbesondere Neurofibromatosen,
- Neuropathien, wie Mangelneuropathien, insbesondere verursacht durch Alkohol, toxische Neuropathien oder durch Arzneimittel verursachte Neuropathien, insbesondere durch Vincristin, Neuropathien, bedingt durch eine metabolische Erkrankung, wie Diabetes, Neuropathien, bedingt durch Entzündungsprozesse, insbesondere das Guillain-Barre-Syndrom, infektiöse Neuropathien, insbesondere Gürtelrose, und Strahlenneuropathien,
- paraneoplastische Polyneuritis,
- multiple Sklerose,
- amyotrophe Lateralsklerose,
- Schizophrenie,
- Depression,
- Gehirntumore,
- die Parkinson-Krankheit und
- Demenz, wie die Alzheimer-Krankheit, die Pick-Krankheit oder vaskuläre Demenz.

4. Verwendung nach einem der Ansprüche 1 bis 3 einer Verbindung mit folgender Formel (II): 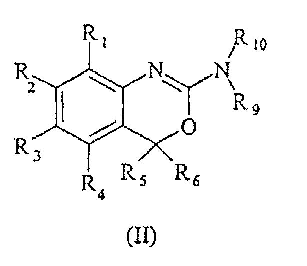 wobei R₁, R₂, R₃, R₄, R₅, R₆, R₉ und R₁₀ wie oben angegeben definiert sind.

5. Verwendung nach einem der Ansprüche 1 bis 4 der Verbindungen mit den folgenden Formeln (III) und (IV):

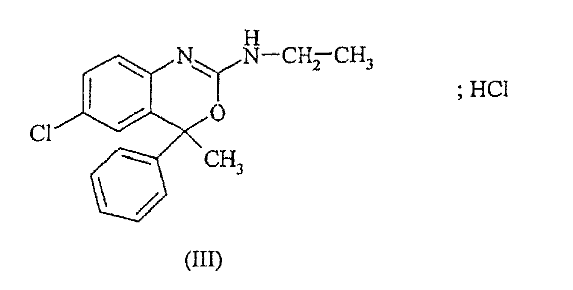

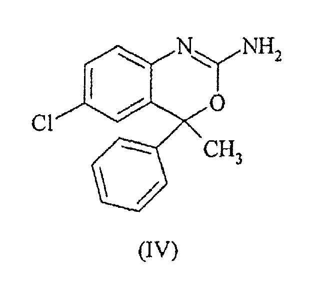

6. Verwendung nach einem der Ansprüche 1 bis 3 einer Verbindung mit folgender Formel (V): 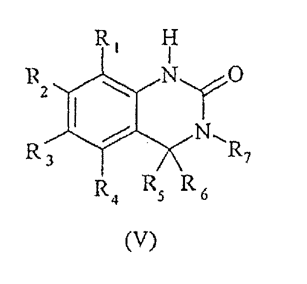 wobei R₁, R₂, R₃, R₅, R₆ und R₇ wie in Anspruch 1 definiert sind.

7. Verwendung nach einem der Ansprüche 1, 2, 3 oder 6 von Verbindungen mit den folgenden Formeln (VI) und (VII):

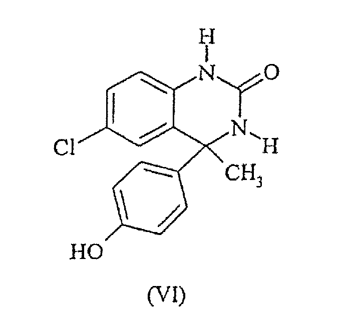

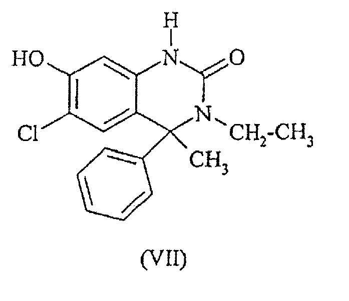

8. Verwendung nach einem der Ansprüche 1 bis 7, wobei das Medikament angepasst ist für die Verabreichung an eine Person, die eine Einheitsdosis zwischen 50 mg und 150 mg, insbesondere zwischen 150 und 200 mg einer Verbindung, wie definiert in einem der Ansprüche 1 und 4 bis 7, benötigt.

9. Verwendung nach einem der Ansprüche 1 bis 8, wobei das Medikament angepasst ist für die Verabreichung an eine Person, die eine Dosis zwischen 50 mg/Tag und 1500 mg/Tag, insbesondere zwischen 150 mg/Tag und 200 mg/Tag, der Verbindung, wie definiert in einem der Ansprüche 1 und 4 bis 7, benötigt.

10. Verwendung nach einem der Ansprüche 1 bis 9, wobei das Medikament für eine orale Verabreichung angepasst ist.

11. Verwendung nach einem der Ansprüche 1 bis 10, wobei das Medikament in der Form eines Pulvers oder in der Form von Tabletten, Kapseln oder Sachets vorliegt.

12. Verwendung nach Anspruch 1, wobei die Verbindung Etifoxin ist und das Medikament einen neurotrophen Effekt ausübt.

13. Verwendung nach einem der Ansprüche 1 bis 11, wobei die Verbindung, wie definiert in einem der Ansprüche 1 und 4 bis 7, mit mindestens einer zusätzlichen Verbindung, bestimmt für die Prävention oder die Behandlung der Pathologien, definiert in Anspruch 3, kombiniert ist.

14. Produkte, enthaltend:
• mindestens eine Verbindung mit Formel (I), wie in einem der Ansprüche 1 und 4 bis 7 definiert, oder ihre pharmazeutisch annehmbaren Salze, und
• mindestens eine zusätzliche Verbindung, bestimmt für die Prävention oder die Behandlung von Pathologien, ausgewählt aus der Liste, umfassend:
- Epilepsie,
- zerebrovaskulären, ischämischen oder hämorrhagischen Insult,
- neurodegenerative Krankheiten, wie die Charcot-Marie-Tooth-Krankheit oder die Friedreich-Krankheit,
- Phakomatosen, insbesondere Neurofibromatosen,
- Neuropathien, wie Mangelneuropathien, insbesondere verursacht durch Alkohol, toxische Neuropathien oder durch Arzneimittel verursachte Neuropathien, insbesondere durch Vincristin, Neuropathien, bedingt durch eine metabolische Erkrankung, wie Diabetes, Neuropathien, bedingt durch Entzündungsprozesse, insbesondere das Guillain-Barre-Syndrom, infektiöse Neuropathien, insbesondere Gürtelrose, und Strahlenneuropathien,
- paraneoplastische Polyneuritis,
- multiple Sklerose,
- amyotrophe Lateralsklerose,
- Schizophrenie,
- Depression,
- Gehirntumore,
- die Parkinson-Krankheit und
- Demenz, wie die Alzheimer-Krankheit, die Pick-Krankheit oder vaskuläre Demenz,
als Kombinationsprodukt zur separaten, gleichzeitigen oder zeitversetzten Verwendung für die Behandlung der neuronalen Schädigungen, die bedingt sind durch diese Pathologien.

15. Verbindung der Formel (I), wie definiert in einem der Ansprüche 1 und 4 bis 7, oder ein pharmazeutisch annehmbares Salz davon, für die Verwendung als Medikament mit einer neuroprotektiven Wirkung bei der Behandlung von neuronalen Schädigungen.

16. Verbindung der Formel (I), oder ein pharmazeutisch annehmbares Salze davon, für die Verwendung nach Anspruch 15 als Medikament mit einer regenerativen Wirkung auf die Zellen des Zentralnervensystems bei der Behandlung von neuronalen Schädigungen.

17. Verbindung der Formel (I), oder ein pharmazeutisch annehmbares Salz davon, für die Verwendung nach Anspruch 15 oder 16, wobei die neuronalen Schädigungen bedingt sind durch Pathologien, ausgewählt aus der Liste, umfassend:
- Epilepsie,
- zerebrovaskulären, ischämischen oder hämorrhagischen Insult,
- neurodegenerative Krankheiten, wie die Charcot-Marie-Tooth-Krankheit oder die Friedreich-Krankheit,
- Phakomatosen, insbesondere Neurofibromatosen,
- Neuropathien, wie Mangelneuropathien, insbesondere verursacht durch Alkohol, toxische Neuropathien oder durch Arzneimittel verursachte Neuropathien, insbesondere durch Vincristin, Neuropathien, bedingt durch eine metabolische Erkrankung, wie Diabetes, Neuropathien, bedingt durch Entzündungsprozesse, insbesondere das Guillain-Barré-Syndrom, infektiöse Neuropathien, insbesondere Gürtelrose, und Strahlenneuropathien,
- paraneoplastische Polyneuritis,
- multiple Sklerose,
- amyotrophe Lateralsklerose,
- Schizophrenie,
- Depression,
- Gehirntumore,
- die Parkinson-Krankheit und
- Demenz, wie die Alzheimer-Krankheit, die Pick-Krankheit oder vaskuläre Demenz.

18. Verbindung der Formel (I), oder ein pharmazeutisch annehmbares Salz davon, für die Verwendung nach einem der Ansprüche 15 bis 17, wobei die Verbindung, wie definiert in einem der Ansprüche 1 und 4 bis 7, mit mindestens einer zusätzlichen Verbindung, bestimmt für die Prävention oder die Behandlung der Pathologien, definiert in Anspruch 17, kombiniert ist.

19. Verbindung der Formel (I), oder ein pharmazeutisch annehmbares Salz davon, für die Verwendung nach Anspruch 15, wobei die Verbindung Etifoxin ist und einen neurotrophen Effekt ausübt.

## Claims

1. Use of at least one compound having the following formula (I): 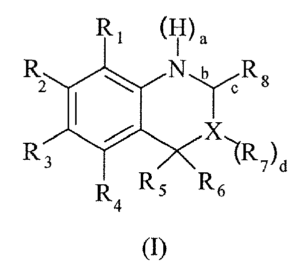 in which:
- a represents 0 or 1,
- b represents a single bond or a double bond,
- c represents a single bond or a double bond,
- d represents 0 or 1,
- X represents an oxygen or nitrogen atom, provided that when X represents an oxygen atom then d has the value of 0 and when X represents a nitrogen atom then d has the value of 1,
- R₁, R₂, R₃, and R₄, which are the same or different, represent a hydrogen atom, a halogen atom, in particular selected from F, Cl, Br, or I, a hydroxyl group, or an alkoxyl group having 1 or 2 carbon atoms,
- R₅ and R₆, which are the same or different, represent a hydrogen atom, an alkyl or cycloalkyl group having from 1 to 6 carbon atoms, or an aryl group having 6 carbon atoms in which the aromatic ring may be substituted by one or more halogen atoms or one or more hydroxyl, alkoxyl having 1 or 2 carbon atoms, trifluoromethyl or nitro groups,
- R₇ represents a hydrogen atom, a hydroxyl group, or an alkyl or hydroxyalkyl group having from 1 to 3 carbon atoms,
- R₈ represents an oxygen atom or a -NR₉R₁₀ group, R₉ and R₁₀, which are the same or different, representing a hydrogen atom, a hydroxyl group, or an alkyl or hydroxyalkyl group having from 1 to 3 carbon atoms, provided than when R₈ represents an oxygen atom then a has the value 1, b represents a single bond and c represents a double bond and that when R₈ represents a -NR₉R₁₀ group then a has the value 0, b represents a double bond and c represents a single bond;
or of its pharmaceutically acceptable salts,
for the manufacture of a medicament having neuroprotective activity which is intended to treat neurone deteriorations.

2. The use according to claim 1, for the manufacture of a medicament having an activity of regeneration of cells of the central nervous system which intended for the treatment of neurone deteriorations.

3. The use according to claim 1 or 2, wherein the neurone deteriorations are linked to diseases selected from the list comprising:
- epilepsy,
- ischaemic or hemorrhagic cerebral vascular accidents,
- neurodegenerative diseases, such as Charcot-Marie-Tooth disease or Friedreich disease,
- phacomatoses, in particular neurofibromatoses,
- neuropathic diseases, such as polyneuropathy, in particular alcoholic polyneuropathy, toxic or drug-induced neuropathy, particularly by vincristine, neuropathy associated with a metabolic disturbance such as diabetes, neuropathy associated with an inflammatory process, in particular Guillain-Barre syndrome, infectious neuropathic diseases, in particular Herpes zoster, and radiation-induced neuropathy,
- paraneoplastic polyneuritis,
- multiple sclerosis,
- amyotrophic lateral sclerosis,
- schizophrenia,
- depression,
- brain tumours,
- Parkinson's disease, and
- dementias, such as Alzheimer's disease, Pick's disease or vascular dementia.

4. The use according to any of claims 1 to 3, of a compound of the following formula (II): 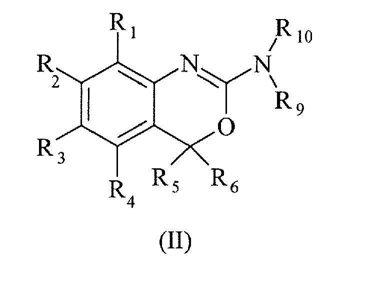 in which R₁, R₂, R₃, R₄, R₅, R₆, R₉ and R₁₀ are as defined above.

5. The use according to any of claims 1 to 4, of compounds of the following formulae (III) and (IV):

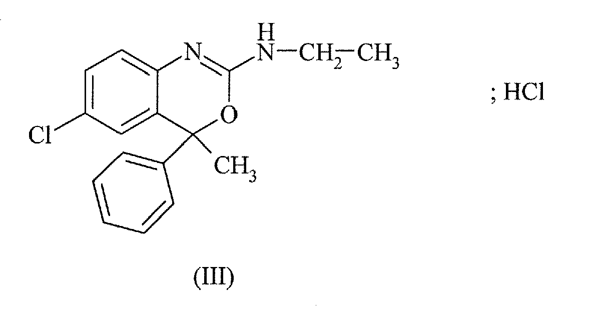

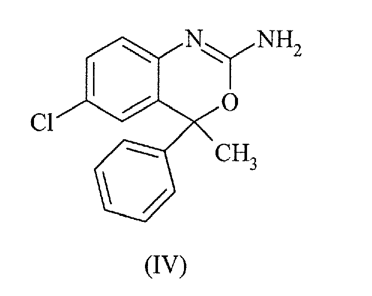

6. The use according to any of claims 1 to 3, of a compound of the following formula (V): 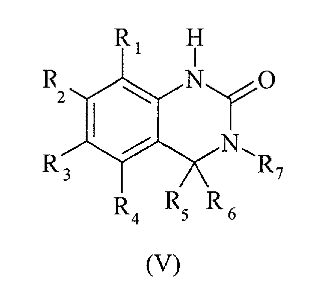 in which R₁, R₂, R₃, R₅, R₆, and R₇ are as defined in claim 1.

7. The use according to claim 1, 2, 3 or 6, of compounds of the following formulae (VI) and (VII):

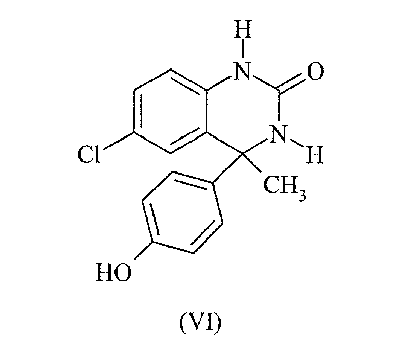

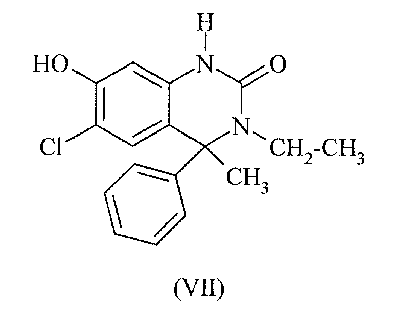

8. The use according to any of claims 1 to 7, wherein the medicament is suitable for administration to an individual in need thereof of a unit dose of from 50 mg to 1500 mg, in particular of from 150 to 200 mg of the compound as defined in any one of claims 1 and 4 to 7.

9. The use according to any of claims 1 to 8, wherein the medicament is suitable for administration to an individual in need thereof of a dose of from 50 mg/d to 1500 mg/d, in particular of from 150 mg/d to 200 mg/d of the compound as defined in any one of claims 1 and 4 to 7.

10. The use according to any of claims 1 to 9, wherein the medicament is suitable for oral administration.

11. The use according to any of claims 1 to 10, wherein the medicament is in the form of a powder, tablet, capsules or sachets.

12. The use according to claim 1, wherein the compound is etifoxine and the medicament has a neurotrophic effect.

13. The use according to any of claims 1 to 11, wherein the compound as defined in any of claims 1 and 4 to 7 is associated to at least one additional compound intended for the prevention or treatment of the diseases defined in claim 3.

14. Products comprising:
• at least one compound of formula (I) as defined in any of claims 1 and 4 to 7 or its pharmaceutically acceptable salts, and
• at least one additional compound intended to prevent or treat diseases selected from the list comprising:
- epilepsy,
- ischaemic or hemorrhagic cerebral vascular accidents,
- neurodegenerative diseases, such as Charcot-Marie-Tooth disease or Friedreich disease,
- phacomatoses, in particular neurofibromatoses,
- neuropathic diseases, such as polyneuropathy, in particular alcoholic polyneuropathy, toxic or drug-induced neuropathy, particularly by vincristine, neuropathy associated with a metabolic disturbance such as diabetes, neuropathy associated with an inflammatory process, in particular Guillain-Barre syndrome, infectious neuropathic diseases, in particular Herpes zoster, and radiation-induced neuropathy,
- paraneoplastic polyneuritis,
- multiple sclerosis,
- amyotrophic lateral sclerosis,
- schizophrenia,
- depression,
- brain tumours,
- Parkinson's disease, and
- dementias, such as Alzheimer's disease, Pick's disease or vascular dementia,
as a combination product for separate, simultaneous or sequential use in the treatment of neurone deteriorations associated with these diseases.

15. A compound of formula (I) as defined in any of claims 1 and 4 to 7, or one of its pharmaceutically acceptable salts, for use as a medicament having a neuroprotective activity in the treatment of neurone deteriorations.

16. A compound of formula (I), or one of its pharmaceutically acceptable salts, for use according to claim 15, as a medicament having an activity of regeneration of cells of the central nervous system in the treatment of neurone deteriorations.

17. A compound of formula (I), or one of its pharmaceutically acceptable salts, for use according to claim 15 or 16, wherein the neurone deteriorations are linked to diseases selected from the list comprising:
- epilepsy,
- ischaemic or hemorrhagic cerebral vascular accidents,
- neurodegenerative diseases, such as Charcot-Marie-Tooth disease or Friedreich disease,
- phacomatoses, in particular neurofibromatoses,
- neuropathic diseases, such as polyneuropathy, in particular alcoholic polyneuropathy, toxic or drug-induced neuropathy, particularly by vincristine, neuropathy associated with a metabolic disturbance such as diabetes, neuropathy associated with an inflammatory process, in particular Guillain-Barre syndrome, infectious neuropathic diseases, in particular Herpes zoster, and radiation-induced neuropathy,
- paraneoplastic polyneuritis,
- multiple sclerosis,
- amyotrophic lateral sclerosis,
- schizophrenia,
- depression,
- brain tumours,
- Parkinson's disease, and
- dementias, such as Alzheimer's disease, Pick's disease or vascular dementia.

18. A compound of formula (I), or one of its pharmaceutically acceptable salts, for use according to any of claims 15 to 17, wherein the compound as defined in any of claims 1 and 4 to 7 is associated to at least one additional compound intended for the prevention or treatment of the diseases defined in claim 17.

19. A compound of formula (I), or one of its pharmaceutically acceptable salts, for use according to claim 15, wherein the compound is etifoxine and it exerts a neurotrophic effect.
